(19) Europäisches Patentamt
European Patent Office
Office européen des brevets

(11) EP 4 733 415 A1

(12) **EUROPEAN PATENT APPLICATION**

(43) Date of publication:
**29.04.2026 Bulletin 2026/18**

(21) Application number: **24209215.3**

(22) Date of filing: **28.10.2024**

(51) International Patent Classification (IPC):
***C12Q 1/6886*** *(2018.01)*

(52) Cooperative Patent Classification (CPC):
**C12Q 1/6886;** C12Q 2600/106; C12Q 2600/118; C12Q 2600/158

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **HOFFMANN, Ralf Dieter**
**Eindhoven (NL)**

(74) Representative: **Philips Intellectual Property & Standards**
**High Tech Campus 52**
**5656 AG Eindhoven (NL)**

Remarks:
The complete document including Reference Table(s) and the Sequence Listing(s) can be downloaded from the EPO website

# (54) A METHOD OF RISK STRATIFICATION FOR LOW RISK PROSTATE CANCER PATIENTS

(57) The invention relates to methods and products for risk stratification of low risk prostate cancer patients. In particular the methods provide for predicting an outcome for prostate cancer patients with an ISUP score of 1 or 2. In addition the invention describes treatment suggestions based on the methods of the invention.

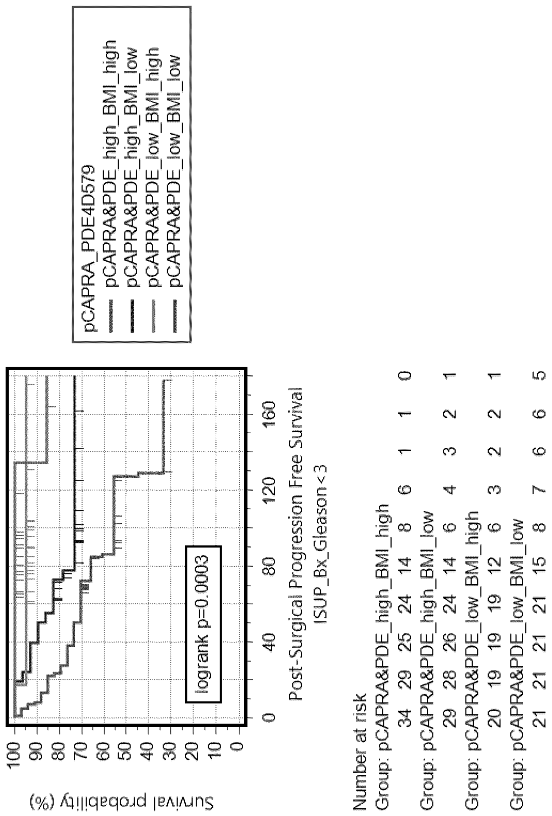


Fig. 16

EP 4 733 415 A1

## Description

FIELD OF THE INVENTION

The invention relates to methods and products for risk stratification of low risk prostate cancer patients. In particular the methods provide for predicting an outcome for prostate cancer patients with an ISUP score of 1 or 2. In addition the invention describes treatment suggestions based on the methods of the invention.

BACKGROUND OF THE INVENTION

Cancer is a class of diseases in which a group of cells displays uncontrolled growth, invasion and sometimes metastasis. These three malignant properties of cancers differentiate them from benign tumors, which are self-limited and do not invade or metastasize. Prostate Cancer (PCa) is the most commonly-occurring non-skin malignancy in men. It displays as a heterogeneous disease with varying potential to develop progressively to deadly forms of the disease. Of the estimated 417,000 annual new cases in Europe, around 92,000 will die from their disease (see Ferlay J. et al., GLOBOCAN 2012 v1.0, Cancer Incidence and Mortality Worldwide: IARC CancerBase No. 11 [Internet], Lyon, France, International Agency for Research on Cancer, 2013).

Clinically, various schemes for pre-surgical risk classification have been developed based upon longitudinal biological patient outcomes (see Rodrigues G. et al., "Pre-treatment risk stratification of prostate cancer patients: A critical review", Canadian Urological Association Journal, Vol. 6, No. 2, pages 121-127, 2012). While active surveillance (AS) is recommended by the various national and international guidelines for men with very low and low risk prostate cancer (see Mohler J. et al., "NCCN clinical practice guidelines in oncology: Prostate cancer, Version 1.2016", Journal of the National Comprehensive Cancer Network, Vol. 14, No. 1, pages 19-30, 2016), there is a significant sub-group in this patient population with a risk of 10 to 25% cancer recurrence after primary treatment (see, for example, Hernandez D.J. et al., "Contemporary evaluation of the D'Amico risk classification of prostate cancer", Journal of Urology, Vol. 70, No. 5, pages 931-935, 2007). These patients suffer from the burden of follow-up treatments that are typically triggered by biochemical relapse. Likewise, in the intermediate risk group there is a sub-population with low risk of biochemical progression (see, for example, Jung J.W. et al., "Stratification of patients with intermediate-risk prostate cancer", BJU International, Vol. 115, No. 6, pages 907-912, 2015). Nevertheless, this group is heterogeneous, comprising patients with varied outcomes, including those with aggressive pathological characteristics (see Abern M.R. et al., "Delayed radical prostatectomy for intermediate-risk prostate cancer is associated with biochemical recurrence: Possible implications for active surveillance from the SEARCH database", The Prostate, Vol. 73, No. 4, pages 409-417, 2013).

WO2019122037A1 describes a method of pre-surgical risk stratification of a prostate cancer subject, comprising determining a gene expression profile for phosphodiesterase 4D variant 7 (PDE4D7) in a biological sample obtained from the subject, determining an expression based risk score for the subject based on the gene expression profile, and determining a pre-surgical prognostic risk score for the subject based on the expression based risk score and pre-surgical clinical variables of the subject.

The typical treatment for low risk prostate cancer patients (ISUP score of 1 or 2) is typically active surveillance, however about 30-50% of this patient population experiences disease progression during active surveillance. Therefor there is a need to provide further risk stratification and treatment options for low risk prostate cancer patients.

These unmet needs are addressed by the methods and product as defined by the appended claims.

SUMMARY OF THE INVENTION

In a first aspect, the invention relates to a method for predicting an outcome for a prostate cancer subject with International Society for Urological Pathology (ISUP) score of 1 or 2, the method comprising determining or receiving the determined expression levels of PDE4D isoforms PDE4D5, PDE4D7 and PDE4D9 in a sample obtained from the subject; receiving the CAPRA score determined for the subject; determining a risk score based on the PDE4D5, PDE4D7 and PDE4D9 expression levels and the Cancer of the Prostate Risk Assessment (CAPRA) score; wherein the method further comprises receiving or determining the Body Mass Index (BMI) of the subject; and determining an outcome for the subject based on the risk score and the BMI; wherein the outcome is a favorable outcome or a non-favorable outcome.

In a second aspect the invention relates to a computer program product comprising instructions which, when the program is executed by a computer, cause the computer to carry out a method comprising: determining a risk score based on received PDE4D5, PDE4D7 and PDE4D9 expression levels and a received CAPRA score, the expression levels being determined in a sample of a prostate cancer subject with ISUP score of 1 or 2, and the CAPRA score obtained from the subject; determining an outcome for the subject based on the risk score and a received BMI from the subject; wherein the outcome is a favorable outcome or a non-favorable outcome.

In a third aspect the invention relates to the use of a kit to determine an outcome for a prostate cancer subject with

ISUP score of 1 or 2, the use comprising using the kit to determine the expression levels of PDE4D isoforms PDE4D5, PDE4D7 and PDE4D9 in sample obtained from the subject; determining a risk score based on the PDE4D5, PDE4D7 and PDE4D9 expression levels and a CAPRA score obtained from the subject; determining an outcome for the subject based on the risk score and a BMI obtained from the subject; wherein the kit comprises at least one primer and/or probe for determining the gene expression profile for each of PDE4D5, PDE4D7 and PDE4D9, and optionally, at least one primer and/or probe for determining the gene expression profile for one or more reference genes selected from the group consisting of: hypoxanthine phosphoribosyl transferase 1 (HPRT1), Tubulin-Alpha- Ib (TUBA1B) pumilio RNA-Binding Family Member (PUM1), and TATA box binding protein (TBP); and optionally, at least one agent for determining a prostate-specific antigen (PSA) level in a biological sample obtained from the subject.

In a fourth aspect the invention relates to a GLP-1 receptor agonist for use in the treatment of prostate cancer in a subject with ISUP score 1 or 2.

BRIEF DESCRIPTION OF THE DRAWINGS

Fig. 1: performance in BCR progression-free survival of the pCAPRA_PDE4D579 model (p -> percentile ranked scores of the CAPRA_PDE4D579 model) in patients with ISUP Gleason score 1 on needle biopsy tissue. The threshold (0.292) was determined from the AUROC (see below). HR and p-value are given.

Fig. 2: performance in BCR progression-free survival of the pCAPRA_PDE4D579 model (p -> percentile ranked scores of the CAPRA_PDE4D579 model) in patients with ISUP Gleason score 1 and 2 on needle biopsy tissue. The threshold (0.292) was determined from the AUROC (see below). HR and p-value are given.

Fig. 3: performance in BCR progression-free survival of the pCAPRA_PDE4D579_BMI model (p -> percentile ranked scores of the CAPRA_PDE4D579_BMI model) in patients with ISUP Gleason score 1 on needle biopsy tissue. The threshold (0.36) was determined from the AUROC (see below). HR and p-value are given.

Fig. 4: performance in BCR progression-free survival of the pCAPRA_PDE4D579_BMI model (p -> percentile ranked scores of the CAPRA_PDE4D579_BMI model) in patients with ISUP Gleason score 1 and 2 on needle biopsy tissue. The threshold (0.36) was determined from the AUROC (see below). HR and p-value are given.

Figs. 5 to 14 performance in BCR progression-free survival of the pCAPRA_PDE4D579 model in patients with ISUP Gleason score 1 on needle biopsy tissue, and BMI>20 (Fig. 5), BMI>21 (Fig. 6), BMI>22 (Fig. 7), BMI>23 (Fig. 8), BMI>24 (Fig. 9), BMI>25 (Fig. 10), BMI>26 (Fig. 11), BMI>27 (Fig. 12), BMI>28 (Fig. 13), and BMI>29 (Fig. 14). The threshold (0.292) was determined from the AUROC (see below). HR and p-value are given.

Fig. 15: performance in BCR progression-free survival of the pCAPRA_PDE4D579 model (p -> percentile ranked scores of the CAPRA PDE4D579 model) in patients with ISUP Gleason score 1 on needle biopsy tissue and pCAPRA_PDE4D579<=0.292 (low) or pCAPRA_PDE4D579>0.292 (high) BMI<=26 (BMI_low) or BMI>26 (BMI_high). HR and p-value are given.

Fig. 16: performance in BCR progression-free survival of the pCAPRA_PDE4D579 model (p -> percentile ranked scores of the CAPRA PDE4D579 model) in patients with ISUP Gleason score 1 and 2 on needle biopsy tissue and pCAPRA_PDE4D579<=0.292 (low) or pCAPRA_PDE4D579>0.292 (high) BMI<=26 (BMI_low) or BMI>26 (BMI_high). HR and p-value are given.

Fig. 17: AUROC analysis of pCAPRA&PDE4D579&BMI model vs the CARP A score alone. The AUC is given in parenthesis. The clinical endpoint is 5-year BCR progression-free survival after surgery.

Fig. 18: AUROC analysis of pCAPRA&PDE4D579&BMI, pCAPRA&PDE4D579, CAPRA Score, and PRIAS models in patients with ISUP Gleason score 1 on needle biopsy tissue. The AUC is given in parenthesis. The clinical endpoint is 5-year BCR progression-free survival after surgery.

Fig. 19: AUROC analysis of pCAPRA&PDE4D579&BMI, pCAPRA&PDE4D579, CAPRA Score, and PRIAS models in patients with ISUP Gleason score 1 and 2 on needle biopsy tissue. The AUC is given in parenthesis. The clinical endpoint is 5-year BCR progression-free survival after surgery.

DEFINITIONS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a

person skilled in the art will understand to still ensure the technical effect of the feature in question. The term typically indicates a deviation from the indicated numerical value of ±20%, preferably ±15%, more preferably ±10%, and even more preferably ±5%.

**"About" and "approximately":** these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

**"Antagonist" and "inhibitor":** These terms are used interchangeably, and they refer to a compound or agent having the ability to reduce or inhibit a biological function of a target protein or polypeptide, such as by reducing or inhibiting the activity or expression of the target protein or polypeptide. Accordingly, the terms "antagonist" and "inhibitor" are defined in the context of the biological role of the target protein or polypeptide. An inhibitor need not completely abrogate the biological function of a target protein or polypeptide, and in some embodiments reduces the activity by at least 20%, 30%, 40%, 50%, 60%, 70%, 80%, 90%, 95%, or 99%. While some antagonists herein specifically interact with (e.g., bind to) the target, compounds that inhibit a biological activity of the target protein or polypeptide by interacting with other members of the signal transduction pathway of which the target protein or polypeptide are also specifically included within this definition. Non-limiting examples of biological activity inhibited by an antagonist include those associated with the development, growth, or spread of a tumor, or an undesired immune response as manifested in autoimmune disease.

**"Anti-cancer effect":** This refers to the effect a therapeutic agent has on cancer, e.g., a decrease in growth, viability, or both of a cancer cell. The IC50 of cancer cells can be used as a measure the anti-cancer effect. IC50 refers to a measure of the effectiveness of a therapeutic agent in inhibiting cancer cells by 50%.

**"Alleviating cancer":** The term, in the context of specific cancers and/or their pathologies, refers to degrading a tumor, for example, breaking down the structural integrity or connective tissue of a tumor, such that the tumor size is reduced when compared to the tumor size before treatment. "Alleviating" metastasis of cancer includes reducing the rate at which the cancer spreads to other organs.

**"Compositions", "products" or "combinations"**: These encompass those compositions suitable for various routes of administration, including, but not limited to, intravenous, subcutaneous, intradermal, subdermal, intranodal, intratumoral, intramuscular, intraperitoneal, oral, nasal, topical (including buccal and sublingual), rectal, vaginal, aerosol and/or parenteral or mucosal application. The compositions, formulations, and products according to the disclosure invention normally comprise the drugs/compound/inhibitor (alone or in combination) and one or more suitable pharmaceutically acceptable excipients or carriers.

**"Combination therapy", or "in combination with":** These term refers to the use of more than one compound or agent to treat a particular disorder or condition. For example, Compound 1 may be administered in combination with at least one additional therapeutic agent. By "in combination with," it is not intended to imply that the other therapy and Compound 1 must be administered at the same time and/or formulated for delivery together, although these methods of delivery are within the scope of this disclosure. Compound 1 can be administered concurrently with, prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks before), or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks after), one or more other additional agents. In general, each therapeutic agent will be administered at a dose and/or on a time schedule determined for that particular agent. The other therapeutic agent can be administered with Compound 1 herein in a single composition or separately in a different composition. Higher combinations, e.g., triple therapy, are also contemplated herein.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at

most" a particular value means that particular value or less. For example, "at most 5" is understood to be the same as "5 or less" i.e., 5, 4, 3, ....-10, -11, etc.

As used herein, the term "and/or" indicates that one or more of the stated cases may occur, alone or in combination with at least one of the stated cases, up to with all of the stated cases.

As used herein, the term "conventional techniques" refers to a situation wherein the methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

As used herein, the term "identity" refers to a measure of the identity of nucleotide sequences or amino acid sequences. In general, the sequences are aligned so that the highest order match is obtained. "Identity" per se has an art-recognized meaning and can be calculated using published techniques. See, e.g.: (Computational Molecular Biology, Lesk, A. M., ED., Oxford University Press, New York, 1988; Biocomputing: Informatics And Genome Projects, Smith, D. W., ED., Academic Press, New York, 1993; Computer Analysis Of Sequence Data, Part I, Griffin, A. M., And Griffin, H. G., EDS., Humana Press, New Jersey, 1994; Sequence Analysis In Molecular Biology, Von Heinje, G., Academic Press, 1987; and Sequence Analysis Primer; Gribskov, M. and Devereux, J., eds., M Stockton Press, New York, 1991). While there exist a number of methods to measure identity between two nucleotide sequences or amino acid sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in Guide To Huge Computers, Martin J. Bishop, ed., Academic Press, San Diego, 1994, and Carillo, H., and Lipton, D., Siam J. Applied Math (1988) 48:1073. Methods to determine identity and similarity are codified in computer programs. Preferred computer program methods to determine identity and similarity between two sequences include, but are not limited to, GCG program package (Devereux, J., et al., Nucleic Acids Research (1984) 12(1):387), BLASTP, BLASTN, FASTA (Atschul, S. F. et al., J. Molec. Biol. (1990) 215:403).

As an illustration, by a polynucleotide having a nucleotide sequence having at least, for example, 95% "identity" to a reference nucleotide sequence encoding a polypeptide of a certain sequence, it is intended that the nucleotide sequence of the polynucleotide is identical to the reference sequence except that the polynucleotide sequence may include up to five point mutations per each 100 nucleotides of the reference amino acid sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: X is intended that the amino acid sequence of the polypeptide is identical to the reference sequence except that the amino acid sequence may include up to five amino acid alterations per each 100 amino acids of the reference amino acid of SEQ ID NO: X. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

The terms sequency identity, sequence homology, and x percent identical to are used herein interchangeably and intend to refer to the same as defined above.

As used herein, the term "in vitro" refers to experimentation or measurements conducted using components of an organism that have been isolated from their natural conditions.

As used herein, the term "ex vivo" refers to experimentation or measurements done in or on tissue from an organism in an external environment with minimal alteration of natural condition.

As used herein, the term "nucleic acid", "nucleic acid molecule" and "polynucleotide" is intended to include DNA molecules and RNA molecules. A nucleic acid (molecule) may be single-stranded or double-stranded, but preferably is double-stranded DNA.

As used herein, the terms "sequence" when referring to nucleotides, or "nucleic acid sequence", "nucleotide sequence" or "polynucleotide sequence" refer to the order of nucleotides of, or within, a nucleic acid and/or polynucleotide. Within the context of the current invention a first nucleic acid sequence may be comprised within or overlap with a further

nucleic acid sequence.

As used herein, the term "subject" or "individual" or "animal" or "patient" or "mammal," used interchangeably, refer to any subject, particularly a mammalian subject, for whom diagnosis, prognosis, or therapy is desired. Mammalian subjects include humans, domestic animals, farm animals, and zoo-, sports-, or pet-animals such as dogs, cats, guinea pigs, rabbits, rats, mice, horses, cattle, cows, bears, and so on. As defined herein a subject may be alive or dead. Samples can be taken from a subject post-mortem, i.e. after death, and/or samples can be taken from a living subject. In a preferred embodiment the subject is a human subject.

In an embodiment the subject has prostate cancer. In an embodiment the prostate cancer subject has an ISUP score of 1 or 2. In an embodiment the subject is pre or post-surgical, meaning radical prostatectomy has been performed is scheduled to be performed in the subject. In an embodiment a diagnostic needle biopsy has been performed on the subject. In an embodiment a CAPRA score is available for the subject. In an embodiment a sample suitable for determining PDE4D5, PDE4D7, and PDE4D9 expression levels, preferably in a tumor cell or a sample comprising a tumor cell, is available, for example in the form of a (diagnostic) needly biopsy or a resected prostate (radical prostatectomy).

As used herein, terms "treatment", "treating", "palliating", "alleviating" or "ameliorating", used interchangeably, refer to an approach for obtaining beneficial or desired results including, but not limited to, therapeutic benefit. By therapeutic benefit is meant eradication or amelioration or reduction (or delay) of progress of the underlying disease being treated. Also, a therapeutic benefit is achieved with the eradication or amelioration or reduction (or delay) of progress of one or more of the physiological symptoms associated with the underlying disease such that an improvement or slowing down or reduction of decline is observed in the patient, notwithstanding that the patient can still be afflicted with the underlying disease.

Herein, the term "outcome" relates to a specific result or effect that can be measured. Examples of an outcome of a subject having melanoma include an outcome of the melanoma, a pathology outcome, an outcome of a therapy for treating melanoma, such as a surgery outcome, a radiation therapy outcome, a chemotherapy outcome, and an immunotherapy outcome, as well as other outcomes, such as a biomarker related, a genomic profile related outcome, an imaging related outcome (e.g., a change in morphology of the tumor), a biology related outcome (e.g., inflammation or immune response), a surrogate marker related outcome, a treatment side effect outcome, a treatment toxicity outcome, a disease pain outcome, a quality of life outcome, a cancer specific survival, and an overall survival.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 4.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence".

The term "progressive prostate cancer state" means that a sample of an individual shows parameter values indicating "biochemical recurrence" and/or "clinical recurrence".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumor cells as measured, for example using in vivo imaging.

The term "prognosticating prostate cancer" as used herein refers to the prediction of the course or outcome of a diagnosed or detected prostate cancer, e.g., during a certain period of time, during a treatment or after a treatment. The term also refers to a determination of chance of survival or recovery from the disease, as well as to a prediction of the expected survival time of a subject. A prognosis may, specifically, involve establishing the likelihood for survival of a subject during a period of time into the future, such as 6 months, 1 year, 2 years, 3 years, 5 years, 10 years or any other period of time.

DETAILED DESCRIPTION OF EMBODIMENTS

The section headings as used herein are for organizational purposes only and are not to be construed as limiting the subject matter described.

A portion of this invention contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent invention, as it appears in the Patent Office patent file or records, but otherwise reserves all copyright rights whatsoever.

Various terms relating to the methods, compositions, uses and other aspects of the present invention are used

throughout the specification and claims. Such terms are to be given their ordinary meaning in the art to which the invention relates, unless otherwise indicated. Other specifically defined terms are to be construed in a manner consistent with the definition as provided herein. The preferred materials and methods are described herein, although any methods and materials similar or equivalent to those described herein can be used in the practice for testing of the present invention. Unless otherwise defined, all technical and scientific terms used herein have the same meaning as commonly understood by a person of ordinary skill in the art.

The invention broadly relates to the realization that the Body Mass Index (BMI) of a prostate cancer patient is predictive for the outcome of prostate cancer patients, particularly in the low risk group of prostate cancer patients. The inventors found that when using a previously described model for risk assessment based on the PDE4D5, PDE4D7 and PDE4D9 gene expression levels combined with the CAPRA score (see e.g. WO 2019/122037, hereby incorporated by reference in its entirety) and supplementing this model with the BMI for the patient, an improved risk stratification can be achieved for patients with an ISUP score of 1 or 2.

Prostate cancer patients with an ISUP score of 1 or 2 are deemed low risk and generally the recommended course of treatment is active surveillance. However about 30-50% of this patient population experiences disease progression during active surveillance. Accurately identifying this subgroup allows for preventive measures such as preventive treatment or additional screening. Earlier described methods based on PDE4D5, PDE4D7 and PDE4D9 expression and CAPRA allow the identification of high risk prostate cancer patients within the entire population (all ISUP risk classes). The inventors herein show that stratification can be significantly improved by including BMI in the model for patients with ISUP score 1 or 2.

In the Experimental data Reference Example 1 and Figs. 1 and 2 show stratification based on the risk score (combination of CAPRA and PDE4D5, PDE4D7 and PDE4D9 expression levels) for ISUP score 1 patients (Fig. 1) and ISUP score 1 or 2 patients (Fig. 2). The Kaplan Meier plot displays progression free survival time. Figures 3 and 4 show stratification based on the risk score (combination of CAPRA and PDE4D5, PDE4D7 and PDE4D9 expression levels) which is combined with BMI for ISUP score 1 patients (Fig. 3) and ISUP score 1 or 2 patients (Fig. 4). Figs. 5-14 show stratification based on the risk score (combination of CAPRA and PDE4D5, PDE4D7 and PDE4D9 expression levels) for patients groups with increasingly high BMI (from BMI>20 in Fig. 5 to BMI>29 in Fig. 14). In Figs. 15 and 16 patients are grouped based on BMI (high or low) and risk score (high or low) and subsequently plotted, the patients are ISUP score 1 (Fig. 15) or ISUP score 1 or 2 (figure 16). AUROC analysis is provided in Figures 17-19, where Fig. 17 compares the BMI / CAPRA / PDE4D5/7/9 model according to the invention with CAPRA only (reference model) in patients not selected for ISUP score, Fig. 18 compares the BMI / CAPRA / PDE4D5/7/9 model according to the invention with reference models CAPRA / PDE4D5/7/9/, CAPRA only or PRIAS only in ISUP score 1 patients; and Fig. 19 compares the BMI / CAPRA / PDE4D5/7/9 model according to the invention with reference models CAPRA / PDE4D5/7/9/, CAPRA only or PRIAS only in ISUP score 1 or 2 patients.

From these data the following can be concluded: current treatment recommendation is based on ISUP and CAPRA score. CAPRA score is a particularly poor predictor in low grade prostate cancer patients (ISUP score 1 or 2, see Figs. 17-19). A better predictor is the previously published model based on CAPRA and PDE4D5/7/9 expression levels (herein referred to as the risk score), however this model can unexpectedly be further improved by including BMI as a parameter in the model. This is demonstrated in Figs. 1-4 where the model of the invention (Figs. 3 and 4) have a better Hazard Ratio compared to the reference model (Figures 1 and 2). The effect of BMI on the prediction can be clearly derived in Figs. 5-14. In addition, Figs. 15 and 16 clearly show a subpopulation of high BMI and high risk score (based on the CAPRA + PDE4D5/7/9 expression levels model), and Figs. 17-19 further emphasize the additional benefit of including BMI in the model particularly for ISUP score 1 prostate cancer patients.

Thus, by assessing the BMI of the patient, the group of patients with a high risk score based on the CAPRA / PDE4D5/7/9 model can be further stratified in two distinct subpopulations where the high BMI / high risk score group has markedly worse outcome than the low BMI high risk score group (see particularly Figs. 15 and 16). This provides for further (preventative) treatment options, where the high BMI / high risk score group would likely benefit from preventative treatment or a more aggressive treatment regime compared to active surveillance.

Therefore, in a first aspect the invention relates to a method for predicting an outcome for a prostate cancer subject with International Society for Urological Pathology (ISUP) score of 1 or 2, the method comprising determining expression levels or receiving determined expression levels of PDE4D isoforms PDE4D5, PDE4D7 and PDE4D9 in a sample obtained from the subject; receiving the Cancer of the Prostate Risk Assessment (CAPRA) score determined for the subject; determining a risk score based on the PDE4D5, PDE4D7 and PDE4D9 expression levels and the CAPRA score; wherein the method further comprises receiving or determining the Body Mass Index (BMI) of the subject; and determining an outcome for the subject based on the risk score and the BMI; wherein the outcome is a favorable outcome or a non-favorable outcome.

The method may be a computer implemented method, thus in an embodiment the invention is a computer implemented method for predicting an outcome for a prostate cancer subject with International Society for Urological Pathology (ISUP) score of 1 or 2, the method comprising receiving the determined expression levels of PDE4D isoforms

PDE4D5, PDE4D7 and PDE4D9 in a sample obtained from the subject; receiving the CAPRA score determined for the subject; determining a risk score based on the PDE4D5, PDE4D7 and PDE4D9 expression levels and the Cancer of the Prostate Risk Assessment (CAPRA) score; wherein the method further comprises receiving or determining the Body Mass Index (BMI) of the subject; and determining an outcome for the subject based on the risk score and the BMI; wherein the outcome is a favorable outcome or a non-favorable outcome.

In an alternative embodiment the method comprises an active step of determining the expression levels and thus the invention relates to a method for predicting an outcome for a prostate cancer subject with International Society for Urological Pathology (ISUP) score of 1 or 2, the method comprising determining the expression levels of PDE4D isoforms PDE4D5, PDE4D7 and PDE4D9 in a sample obtained from the subject; receiving the CAPRA score determined for the subject; determining a risk score based on the PDE4D5, PDE4D7 and PDE4D9 expression levels and the Cancer of the Prostate Risk Assessment (CAPRA) score; wherein the method further comprises receiving or determining the Body Mass Index (BMI) of the subject; and determining an outcome for the subject based on the risk score and the BMI; wherein the outcome is a favorable outcome or a non-favorable outcome.

Body mass index (BMI) is a value derived from the mass (weight) and height of a person. The BMI is defined as the body mass divided by the square of the body height, and is expressed in units of kg/m2, resulting from mass in kilograms (kg) and height in meters (m). The BMI is a convenient rule of thumb used to broadly categorize a person as based on tissue mass (muscle, fat, and bone) and height. Major adult BMI classifications are underweight (under 18.5 kg/m2), normal weight (18.5 to 24.9), overweight (25 to 29.9), and obese (30 or more).

In an embodiment the BMI is the BMI that has been determined for the prostate cancer patient at the time of taking a biopsy and/or at the time of prostate surgery, such as partial or total removal of the prostate (prostatectomy).

In an embodiment a threshold BMI is set to distinguish between "high" BMI and "low" BMI. For example, the threshold BMI may be set as an integer such as 24, 25, 26, 27, 28, 29, or 30, preferably 25, 26, 27, 28, or 29, or it may be any number including fractions in the range of 24 to 30, preferably 25 to 29. Using conventional techniques a suitable threshold may be determined by analyzing a reference dataset of ISUP score 1 or 2 prostate cancer patients and their respective BMI.

Treatment decisions in primary, localized prostate cancer are largely subject to a combination of the risk of future disease progression and life expectancy. The National Comprehensive Cancer Network (NCCN) has defined five risk categories based on pre-surgical clinical variables (see Mohler J. et al.). For each risk group, ranging from very low, low, intermediate (dichotomized into favorable vs. unfavorable intermediate), high and very high risk, several options of interventions are presented in current practice prostate cancer guidelines. More advanced tools of clinical risk prediction have been presented in the past in the form of mathematical models, which combine the value of clinical variables into a single score (see Lughezani G. et al., "Predictive and prognostic models in radical prostatectomy candidates: A critical analysis of the literature". European Urology, Vol. 58, No. 5, pages 687-700, 2010). One of the most extensively validated clinical risk algorithms for pre-surgical decision support is the pre-surgical CAPRA score (see Cooperberg M.R., "The UCSF Cancer of the Prostate Risk Assessment (CAPRA) Score: A straightforward and reliable pre-operative predictor of disease recurrence after radical prostatectomy", Journal of Urology, Vol. 173, No. 6, pages 1938-1942, 2005). The score is a combination of clinically available information, i.e., patient age, pre-operative PSA, biopsy Gleason, percentage of tumor positive biopsies, and clinical stage. Initially published in 2005, this score has been validated in several studies since then (see Brajtbord J.S. et al., "The CAPRA score at 10 years: Contemporary perspectives and analysis of supporting studies", European Urology, Vol. 71, No. 5, pages 705-709, 2017). By combining the molecular information provided by the expression based risk score with the information from such extensively validated pre-surgical clinical variables, a pre-surgical prognostic risk score with an improved prognostic power may be obtained.

Thus the CAPRA provides a straightforward score from 0 to 10 based on the following parameters:

Age at diagnosis (0 points for <50, 50 and older 1 point)
PSA levels at diagnosis (less or equal than 6 ng/mL: 0; between 6.1 and 10 ng/mL: 1; between 10.1 and 20 ng/mL: 2; between 20.1 and 30 ng/mL: 3; and more than 30 ng/mL: 4 points)
Gleason score of the biopsy (primary/secondary) no pattern 4 or 5: 0 points; secondary pattern 4 or 5: 1 point; and primary pattern 4 or 5: 3 points,
Clinical stage (T stage): T1 or T2: 0 points; T3a: 1 point
Percent of biopsy cores involved with cancer (positive for cancer): less than 34%: 0 points; 34% or more: 1 point.

In an embodiment the method comprises determining the CAPRA score for the prostate cancer patient. In an embodiment the received or determined CAPRA score is a CAPRA score that has been determined for the prostate cancer patient at the time of taking a biopsy and/or at the time of prostate surgery, such as partial or total removal of the prostate (prostatectomy). Thus, in an embodiment the risk score is based on clinical variables. In an embodiment the clinical variables comprise one or more of: (i) an age of the subject; (ii) a prostate-specific antigen (PSA) level; (iii) a primary and secondary biopsy Gleason score; (iv) a clinical stage; and (v) a percentage of tumor positive biopsies.

In an embodiment in the favorable outcome is low chance of post-surgical disease progression and wherein the non-favorable outcome is high chance of post-surgical disease progression.

When used herein the term favorable refers to a situation which is desirable with respect to an outcome for a patient with prostate cancer. For example, the term may indicate the absence or low chance of biochemical recurrence or cancer related death. Similarly, the term unfavorable refers to a situation which is not desirable with respect to an outcome for a patient with prostate cancer, such as but not limited to the occurrence of or an increased chance of developing biochemical recurrence, or cancer related death. In an embodiment e favorable outcome is low chance of post-surgical disease progression and wherein the non-favorable outcome is high chance of post-surgical disease progression. For example, a favorable outcome (low chance of post-surgical disease progression) may indicate a chance of 40% or less, preferable 35%, 30%, 25% or even 20% or less chance of post-surgical disease progression within 150 months after diagnosis. An unfavorable outcome (high chance of post-surgical disease progression) may indicate a chance of 40% or more, preferable 45%, 50%, 55% or even 60% or more chance of post-surgical disease progression within 150 months after diagnosis.

In an embodiment the subject is a human subject. In an embodiment the subject is a post-surgical prostate cancer patient or a pre-surgical prostate cancer patient. When used herein "post-surgical prostate cancer patient" refers to a prostate cancer patient having undergone partial or full removal of the prostate (radical prostatectomy), or orchidectomy, or a combination thereof. When used herein "pre-surgical prostate cancer patient" refers to a prostate cancer patient scheduled to undergo partial or full removal of the prostate (radical prostatectomy), or orchidectomy, or a combination thereof.

In an embodiment the sample is a sample obtained from the subject, wherein the sample comprises at least one tumor cell. The term "sample" when used herein is used interchangeably with the term "biological sample" and may refer to any biological material containing or comprising tumor cells or nucleic acids derived from tumor cells, obtained via suitable methods known to the person skilled in the art from a subject, e.g., a prostate cancer patient. The sample used may be collected in a clinically acceptable manner, e.g., in a way that nucleic acids (in particular RNA) or proteins are preserved. The sample may include body tissue and/or a fluid, such as, but not limited to, blood, sweat, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumor cells, extracellular vesicles, a sample containing prostate secreted exosomes, or cell lines or cancer cell line. In an embodiment the sample is a tumor sample, a blood sample, a urine sample, or a biopsy such as a needle biopsy. As a needle biopsy is needed to determine a CAPRA score preferable the needly biopsy is also the sample. In an embodiment the sample is a sample obtained by a needle biopsy or the sample is obtained from the resected prostate.

In one particular realization, biopsy or resections samples may be obtained and/or used. Such samples may include cells or cell lysates. It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below. Furthermore, cells, e.g., tumor cells, may be enriched via filtration processes of fluid or liquid samples, e.g., blood, urine, etc. Such filtration processes may also be combined with enrichment steps based on ligand specific interactions as described herein above.

Determining the expression levels of the PDE4D isoforms may be performed using methods known in the art. For example, the gene expression levels may be determined by detecting mRNA expression using one or more primers and/or probes and/or one or more sets thereof. Moreover, the gene expression profile may be determined by an amplification based method and/or microarray analysis and/or RNA sequencing. The determining of the gene expression profile may include performing Real-Time Quantitative Polymerase Chain Reaction (RT-qPCR) on RNA extracted from the biological sample. In other embodiments, the gene expression profile is determined by RNA sequencing, conventional PCR (using, e.g., end point analysis by gel electrophoresis), or multiplex-PCR. In the case of RT-qPCR, the determining of the gene expression profile may include determining a threshold cycle (Ct) value for PDE4D7 and each of the one or more reference genes. The PCR may be performed with at least one primer and/or probe for measuring a reference gene selected from HPRT1, TUBA1B, PUM1, and TBP.

Preferably the expression levels, e.g. the PDE4D5, PDE4D7 and PDE4D9 expression levels have been or are determined for the prostate cancer patient at the time of taking a biopsy and/or at the time of prostate surgery, such as partial or total removal of the prostate (prostatectomy). Thus ideally the expression levels are determined in a sample obtained during biopsy or prostatectomy. Thus the sample may be taken from the biopsy or resected prostate, but may also be a separate sample as described herein.

The International Society of Urological Pathology (ISUP) Grade is a newer, easier to understand and more accurate system that replaces the Gleason score, and is used for predicting how quickly your cancer might spread. It is done using prostate biopsy samples. The ISUP Grade uses a 1 to 5 grade group system. The higher the grade group, the higher the risk of your cancer being aggressive and spreading rapidly.
ISUP score 1 corresponds to Gleason grade 3+3=6 and represents a low risk: the cancer is usually slow growing and less likely to spread;
ISUP score 2 corresponds to Gleason grade 3+4=7 and represents an intermediate favorable risk: the cancer can be moderately likely to spread;
ISUP score 3 corresponds to Gleason grade 4+3=7 and represents an intermediate unfavorable risk: the cancer can be moderately likely to spread;
ISUP score 4 corresponds to Gleason grade 4+4=8 and represents a high risk: the cancer can be fast growing and more likely to spread;
ISUP score 5 corresponds to Gleason grade 9 or 10 and represents the highest risk: the cancer can be fast growing and most likely to spread;
The ISUP score is based on the Gleason scoring system and can be determined using methods known in the art, and for example described in Epstein et al., Contemporary Prostate Cancer Grading System: A Validated Alternative to the Gleason Score. Eur Urol. 2016 Mar;69(3):428-35, hereby incorporated by reference in its entirety. Briefly the Gleason score determines cell abnormality in two distinct areas on a scale from 1 to 5.
When used herein the term "outcome" refers to a prostate cancer related outcome. The outcome may for example refer to time to death, time to cancer related death, time to biochemical recurrence or time to metastasis. The predicted outcome may be expressed as chance that the outcome will occur or not occur. Such predicted chance may be set for a predetermined time span. For example the outcome may be chance of biochemical recurrence within 5 years. In this context a favorable or good outcome or an unfavorable or bad outcome can be
The method described herein determines an outcome for a prostate cancer patient based on combining the information of the BMI of the patient and the risk score of the patient. In its simplest form these two factors are simply evaluated separately, that is the risk score (as defined herein) is calculated and the BMI is determined, and based on these two parameters the outcome is determined. The outcome may be a favorable (also referred to as good) outcome or a unfavorable (also referred to as poor) outcome. A high risk score indicates an unfavorable outcome and a low risk indicates a favorable outcome. In addition a high BMI indicates an even further unfavorable outcome in the high risk score group compared to a low BMI, but BMI does not significantly correlate with outcome for patients in the low risk score group. These findings have implications for potential treatment or prevention strategies, particularly for the group of patients with high risk score and high BMI.
In an embodiment the risk score and BMI are combined with a logistic regression model. In an embodiment the BMI, CAPRA score and PDE4D5, PDE4D7, and PDE4D9 are combined with a logistic regression model. In an embodiment the outcome is based on a score calculated from the BMI and risk score. For example the score may be calculated as:

$$SCORE = a * BMI + b * Risk_Score + c$$

wherein each of a, b and c are constants.
The Risk_Score may for example be determined as:

$$Risk_Score = d * CAPRA + e * expression_score + f$$

wherein each of d, e, and f are constants.
The expression score may be calculated as:

$$expression_score - g * PDE4D5 + h * PDE4D7 + i * PDE4D9 + j$$

wherein each of g, h, i, and j are constants and each of PDE4D5, PDE4D7 and PDE4D9 are a numerical value representing the respective expression levels, preferably normalized expression levels, of the respective PDE4D isoforms.
The SCORE may be simplified to:

$$SCORE = k * BMI + l * CAPRA + m * PDE4D5 + n * PDE4D7 + o * PDE4D9 + p$$

wherein each of k, 1, m, n, o, and p are constants.
When used herein the term "expression based risk score" is used to indicate a numerical value based on the

combined PDE4D5, PDE4D7 and PDE4D9 expression levels. Thus the expression based risk score may be calculated using the expression above for "expression score", by adding up the expression levels individually multiplied by constant and optionally adding a constant. Preferably the expression levels are normalized, more preferably the expression levels are normalized using one or more of the reference genes HPRT1, TUBA1B, PUM1, and/or TBP.

The gene expression profile includes expression information from phosphodiesterase 4D variant 5 (PDE4D5), phosphodiesterase 4D variant 7 (PDE4D7) and from phosphodiesterase 4D variant 9 (PDE4D9), wherein an expression based risk score is determined for the subject for each of the phosphodiesterase 4D variants based on the gene expression profile, and wherein the risk score for the subject is determined based on the expression based risk scores and the CAPRA score of the subject. Like the PDE4D7 isoform, the PDE4D5 and PDE4D9 isoforms are long isoforms that each contain both the UCR1 and UCR2 regulatory domains. Thus the risk score as defined herein is based on information from the gene expression profiles for PDE4D5, PDE4D7, and PDE4D9 in the CAPRA score in a combination model. Such model allows to predict longitudinal clinical outcomes for prostate cancer patients.

It is particularly preferred that the determining of the gene expression profile comprises performing RT-qPCR on RNA extracted from the biological sample, wherein a Cq value is determined for each of PDE4D5, PDE4D7, and PDE4D9 and for each of the one or more reference genes, and wherein the determining of the expression based risk score includes normalizing the Cq values for PDE4D5, PDE4D7, and PDE4D9 using the Cq value for each of the one or more reference genes and computing the expression based risk score as a linear function of the normalized Cq value.

For example, the normalized Cq value for PDE4D7 may be generated by applying the following:

$$N(Cq_{PDE4D7}) = Mean(Cq_{ref_genes}) - (Cq_{PDE4D7}), \qquad (2)$$

where $N(Cq_{PDE4D7})$ is the normalized genes expression profile value (quantification cycle, Cq) of PDE4D7, $Mean(Cq_{ref_genes})$ is the arithmetic mean of the PCR Cq values of the one or more reference gene, and $Cq_{PDE4D7}$ is the PCR Cq value of PDE4D7. The same can be applied to PDE4D5 and PDE4D9.

The term "phosphodiesterase 4D5" or "PDE4D5" refers to the splice variant 5 of the human phosphodiesterase PDE4D, i.e., the human phosphodiesterase PDE4D5 gene, for example, to the sequence as defined in NCBI Reference Sequence: NM_001197218.1, specifically, to the nucleotide sequence as set forth in SEQ ID NO: 12, which corresponds to the sequence of the above indicated NCBI Reference Sequence of the PDE4D5 transcript, and also relates to the corresponding amino acid sequence for example as set forth in SEQ ID NO: 13, which corresponds to the protein sequence defined in NCBI Protein Accession Reference Sequence NP_001184147.1 encoding the PDE4D5 polypeptide. The term "phosphodiesterase 4D5" or "PDE4D5" also relates to the amplicon that can be generated by the primer pair PDE4D5forward (SEQ ID NO:14) and the PDE4D5_reverse (SEQ ID NO:15) and can be detected by probe SEQ ID NO:16.

The term "PDE4D5" also comprises nucleotide sequences showing a high degree of homology to PDE4D7, e.g., nucleic acid sequences being at least 75%, 80%, 85%, 90%, 91%, 92%, 93%, 94%, 95%, 96%, 97%, 98% or 99% identical to the sequence as set forth in SEQ ID NO:12 or amino acid sequences being at least 75%, 80%, 85%, 90%, 91%, 92%, 93%, 94%, 95%, 96%, 97%, 98% or 99% identical to the sequence as set forth in SEQ ID NO:13 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:13 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:12.

The term "phosphodiesterase 4D7" or "PDE4D7" refers to the splice variant 7 of the human phosphodiesterase PDE4D, i.e., the human phosphodiesterase PDE4D7 gene, for example, to the sequence as defined in NCBI Reference Sequence: NM_001165899.1, specifically, to the nucleotide sequence as set forth in SEQ ID NO: 19, which corresponds to the sequence of the above indicated NCBI Reference Sequence of the PDE4D7 transcript, and also relates to the corresponding amino acid sequence for example as set forth in SEQ ID NO:20, which corresponds to the protein sequence defined in NCBI Protein Accession Reference Sequence NP_001159371.1 encoding the PDE4D7 polypeptide. The term "phosphodiesterase 4D7" or "PDE4D7" also relates to the amplicon that can be generated by the primer pair PDE4D7_forward (SEQ ID NO:21) and the PDE4D7_reverse (SEQ ID NO:22) and can be detected by probe SEQ ID NO:23. The PDE4D7 polypeptide can also be detected with primer pair PDE4D7-2_forward (SEQ ID NO:24) and the PDE4D7_reverse (SEQ ID NO:25) and can be detected by probe SEQ ID NO:26.

The term "PDE4D7" also comprises nucleotide sequences showing a high degree of homology to PDE4D7, e.g., nucleic acid sequences being at least 75%, 80%, 85%, 90%, 91%, 92%, 93%, 94%, 95%, 96%, 97%, 98% or 99% identical to the sequence as set forth in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:19.

The term "phosphodiesterase 4D9" or "PDE4D9" relates to the splice variant 9 of the human phosphodiesterase PDE4D, i.e., the human phosphodiesterase PDE4D9 gene, for example, to the sequence as defined in NCBI Reference Sequence: NM_001197220.1, specifically, to the nucleotide sequence as set forth in SEQ ID NO:29, which corresponds to the sequence of the above indicated NCBI Reference Sequence of the PDE4D9 transcript, and also relates to the corresponding amino acid sequence for example as set forth in SEQ ID NO:30 which corresponds to the protein sequence defined in NCBI Protein Accession Reference Sequence NP_001184149.1 encoding the PDE4D9 polypeptide. The term "phosphodiesterase 4D9" or "PDE4D9" also relates to the amplicon that can be generated by the primer pair PDE4D9_forward (SEQ ID NO:31) and the PDE4D9_reverse (SEQ ID NO:32) and can be detected by probe SEQ ID NO:33.

The term "PDE4D9" also comprises nucleotide sequences showing a high degree of homology to PDE4D7, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or amino acid sequences being at least 75%, 80%, 85%, 90%, 91%, 92%, 93%, 94%, 95%, 96%, 97%, 98% or 99% identical to the sequence as set forth in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29.

The reference genes are defined by the sequences set out below, and their expression levels may be determined using the primer probe sets indicated therein. The skilled person is aware that other prime probe sets or non (quantitative) PCR based techniques may be used to determine expression levels, such as but not limited to RNA sequencing or microarray.

*TABLE 1: Exemplary primer and probe nucleic acid sequences of the reference genes.*

| Gene Name | Exemplary NCBI RefSeq | Exemplary Protein Accession | Sense Primer | Antisense primer | Probe Sequence |
|---|---|---|---|---|---|
| HPRT1 | NM_00019 4.2 (SEQ ID NO: 34) | NP_00018 5.1 (SEQ ID NO: 35) | GAGGATTTG GAAAGGGT GTTTATT (SEQ ID NO:36) | ACAGAGG GCTACAAT GTGATG (SEQ ID NO:37) | ACGTCTTGCT CGAGATGTGA TGAAGG (SEQ ID NO:38) |
| TUBA1B | NM_00608 2.2 (SEQ ID NO: 39) | NP_00607 3.2 (SEQ ID NO: 40) | TGACTCCTT CAACACCTT CTTC (SEQ ID NO:41) | TGCCAGTG CGAACTTC AT (SEQ ID NO:42) | CCGGGCTGTG TTTGTAGACT TGGA (SEQ ID NO:43) |
| PUM1 | NM_00102 0658.1 (SEQ ID NO: 44); NM_01467 6.2 (SEQ ID NO:45) | NP_00101 8494.1 (SEQ ID NO: 46); NP_05549 1.1 (SEQ ID NO:47) | GCCAGCTTG TCTTCAATG AAAT (SEQ ID NO:48) | CAAAGCC AGCTTCTG TTCAAG (SEQ ID NO:49) | ATCCACCATG AGTTGGTAGG CAGC (SEQ ID NO:50) |
| TBP | NM_00319 4.4 (SEQ ID NO: 51) | NP_00318 5.1 (SEQ ID NO: 52) | GCCAAGAA GAAAGTGA ACATCAT (SEQ ID NO:53) | ATAGGGAT TCCGGGAG TCAT (SEQ ID NO:54) | TCAGAACAAC AGCCTGCCAC CTTA (SEQ ID NO:55) |

(continued)

| Gene Name | Exemplary NCBI RefSeq | Exemplary Protein Accession | Sense Primer | Antisense primer | Probe Sequence |
|---|---|---|---|---|---|
| ACTB | NM_00110 1.3 SEQ ID NO: 56) | NP_00109 2.1 (SEQ ID NO: 57) | CCAACCGCG AGAAGATG A (SEQ ID NO:58) | CCAGAGG CGTACAGG GATAG (SEQ ID NO:59) | CCATGTACGT TGCTATCCAG GCT (SEQ ID NO:60) |
| RPLP0 | NM_00100 2.3 (SEQ ID NO:61) | NP_44450 5.1/NP_00 0993.1 (SEQ ID NO: 62/63) | TAAACCCTG CGTGGCAAT (SEQ ID NO:64) | ACATTTCG GATAATCA TCCAATAG TTG (SEQ ID NO:65) | AAGTAGTTGG ACTTCCAGGT CGCC (SEQ ID NO:66) |
| ALAS-1 | NM_00068 8.5/NM_19 9166.2 (SEQ ID NO:67/68) | NP_00067 9.1/NP_95 4635.1 (SEQ ID NO:69/70) | AGCCACATC ATCCCTGT SEQ ID NO:71) | CGTAGATG TTATGTCT GCTCAT (SEQ ID NO:72) | TTTAGCAGCA TCTGCAACCC GC (SEQ ID NO:73) |

The method further comprises:
determining a pre-surgical Cancer of the Prostate Risk Assessment (CAPRA) score for the subject,
wherein the pre-surgical prognostic risk score is determined by combining the expression based risk score and the pre-surgical CAPRA score.

As mentioned above, the pre-surgical CAPRA score is one of the most extensively validated clinical risk algorithm for pre-surgical decision support in prostate cancer. It provides a categorical score between 1 and 10 with three categories of low risk (pre-surgical CAPRA scores 0 to 2), intermediate risk (pre-surgical CAPRA scores 3 to 5), and high risk (pre-surgical CAPRA scores 6 to 10). In view of its current level of validation as a prognostic algorithm in prostate cancer as well as its easy-to-interpret single score output, the pre-surgical CAPRA score can advantageously be combined with the expression based risk score into a pre-surgical prognostic risk score that may easily be determined in clinical practice and that may allow for a further improvement in pre-surgical prognosis compared to the use of the pre-surgical CAPRA algorithm alone.

When used herein the term CAPRA score is the CAPRA score determined for the prostate patient at the time of determining the relevant parameters on which the CAPRA score is based. The score is typically based on a needle biopsy, among others. As a diagnostic needle biopsy is typically taken pre-surgery (e.g. radical prostatectomy), the CAPRA score may be a pre-surgical CAPRA score.

It is further preferred that the expression based risk score and the pre-surgical CAPRA score are combined with a regression function derived from a population of prostate cancer subjects.

Regression analysis helps one understand how the typical value of the dependent variable (or "criterion variable") changes when any one of the independent variables is varied, while the other independent variables are held fixed. This relationship between the dependent variable and the independent variables is captured in the regression function, which can be used to predict the dependent variable given the values of the independent variables. The dependent variable can be, for example, a binary variable, such as biochemical relapse within 5 years after surgery. In this case, the regression is a logistic regression that is based on a logit function of the independent variables, which, here, comprise or consist of the expression based risk score and the pre-surgical CAPRA score. By means of the regression function, an improved prediction of e.g. the 5-year risk of biochemical recurrence after surgery may be possible.

In an alternative, it is preferred that the pre-surgical prognostic risk score is determined as a modified pre-surgical Cancer of the Prostate Risk Assessment (CAPRA) score for the subject, in which a primary and secondary biopsy Gleason

score is replaced by the expression based risk score.

The biopsy Gleason score has, on one hand, a significant impact to the pre-surgical CAPRA score. At the same time, however, it was found that Gleason scoring is subject to substantial variability amongst pathologists. By replacing the information of the biopsy Gleason score within the CAPRA score with the molecular information provided by the expression based risk score, a modified CAPRA score may be obtained that can be more reliable and less susceptible to variations in the assessment provided by different pathologists.

It is further preferred that the expression based risk score is a value in a predefined range, wherein depending on the value a number of points in the range from 0 to 3 are added in the modified pre-surgical CAPRA score.

The primary and secondary biopsy Gleason score is considered in the pre-surgical CAPRA score as follows: If both the primary and secondary biopsy Gleason score are in the range of 1 to 3, no point is added in the modified pre-surgical CAPRA score. Alternatively, if the primary biopsy Gleason score is in the range of 1 to 3 and the secondary biopsy Gleason score is in the range of 4 to 5, one point is added in the modified pre-surgical CAPRA score. Finally, if the primary biopsy Gleason score is in the range of 4 to 5 and the secondary biopsy Gleason score is in the range of 1 to 5, three points are added in the modified pre-surgical CAPRA score. By adding, depending on the value of the expression based risk score, a number of points in the range from 0 to 3 in the modified pre-surgical CAPRA score, the overall structure of the resulting modified pre-surgical CAPRA score can be kept the same with a minimum total score of 0 and a maximum total score of 10.

In one preferred example, the expression based risk score is a value in the range of 1 to 5 and three points are added in the modified pre-surgical CAPRA score if the value is in the range of 1 to <2, whereas two points are added if the value is in the range of 2 to <3, one point is added if the value is in the range of 3 to <4, and no point is added if the value is in the range of 4 to <5.

It is further preferred that the method comprises:

normalizing the gene expression profile with respect to one or more reference genes selected from the group consisting of: hypoxanthine phosphoribosyltransferase 1 (HPRT1), Tubulin-Alpha-1b (TUBA1B), pumilio RNA-Binding Family Member (PUM1), and TATA box binding protein (TBP),
wherein the expression based risk score is determined based on the normalized gene expression profile.

By normalizing the gene expression profile with respect to one or more reference genes and by determining the expression based risk score is determined based on the normalized gene expression profile, variability in the determination of the expression based risk score can be reduced. This enables differentiation between real variations in gene expression profiles and variations due to the measurement processes. In this respect, it has been found that HPRT1, TUBA1B, PUM1, and TBP are particularly well suited as reference genes for normalizing the PDE4D7 gene expression profile.

The gene expression profile may be determined by detecting mRNA expression using one or more primers and/or probes and/or one or more sets thereof. Moreover, the gene expression profile may be determined by an amplification based method and/or microarray analysis and/or RNA sequencing. The determining of the gene expression profile may include performing Real-Time Quantitative Polymerase Chain Reaction (RT-qPCR) on RNA extracted from the biological sample. In other embodiments, the gene expression profile is determined by RNA sequencing, conventional PCR (using, e.g., end point analysis by gel electrophoresis), or multiplex-PCR. In the case of RT-qPCR, the determining of the gene expression profile may include determining a threshold cycle (Ct) value for PDE4D7 and each of the one or more reference genes. The PCR may be performed with at least one primer and/or probe for measuring a reference gene selected from HPRT1, TUBA1B, PUM1, and TBP.

It is preferred that the one or more reference genes comprise at least two, or at least three of HPRT1, TUBA1B, PUM1, and TBP. In a particularly preferred realization, the one or more reference genes comprise all of HPRT1, TUBA1B, PUM1, and TBP.

Other reference genes which may be additionally or alternatively used for normalizing the PDE4D7 gene expression profile include: actin, beta, mRNA (ACTB); 60S acidic ribosomal phosphoprotein P0 mRNA (RPLPO); Polymerase (RNA) II (DNA Directed) Polypeptide A, 220kDa (POLR2A); Beta-2-Microglobulin (B2M); and Aminolevulinate-Delta-Synthase (ALAS-1).

It is further preferred that the expression based risk score is determined with a scoring function, based on the gene expression profile, the scoring function having been derived from gene expression profiles for biological samples of prostate cancer subjects.

Herein, it is particularly preferred that the scoring function is based on the normalized gene expression profile, e.g., the gene expression profile normalized with respect to all of HPRT1, TUBA1B, PUM1, and TBP, and that the scoring function is derived from correspondingly normalized gene expression profiles for biological samples of prostate cancer subjects. In one preferred realization, the scoring function is a linear transformation that transforms the normalized gene expression profile into a predefined range of values, such as the above-mentioned range of 1 to 5. Such a transformation

can be determined by considering the frequency distribution of the normalized gene expression profile values for PDE4D5, PDE4D7, and PDE4D9 for biological samples of a population of prostate cancer subjects and by determining the transformation that transforms the frequency distribution into the desired range. By making use of such a scoring function, the expression based risk score can be expressed in a way that is intuitive to a user, such as in a small positive value range. This is similar to other categories used in the clinical routine, e.g., in histo-pathology grading (Gleason) or multi-parametric MRI radiology scoring (PIRADS).

Thus in an embodiment the PDE4D5, PDE4D7 and PDE4D9 expression levels and the CAPRA score are combined to obtain a risk score using a regression function derived from a population of prostate cancer subjects. In an embodiment the risk score is a modified CAPRA score for the subject, wherein the PDE4D5, PDE4D7 and PDE4D9 expression levels used to calculate the modified CAPRA score are combined to a value representing an expression score in a predefined range, wherein depending on the value a number of points in the range from 0 to 3 are added to the CAPRA score to obtain the modified CAPRA score. In an embodiment the value representing the expression score is determined with a scoring function, based on the PDE4D5, PDE4D7 and PDE4D9 expression level, the scoring function having been derived from gene expression profiles for biological samples of prostate cancer subjects.

In a further embodiment the PDE4D5, PDE4D7 and PDE4D9 expression levels are normalized using one or more of the reference genes hypoxanthine phosphoribosyl transferase 1 (HPRT1), Tubulin- Alpha- lb (TUBA1B) pumilio RNA-Binding Family Member (PUM1), and TATA box binding protein (TBP).

In an embodiment the method further comprises proposing a primary treatment for the subject based on the determined outcome. In an embodiment the recommended treatment is: active surveillance with de-escalation of monitoring when the risk score is low and the BMI is low or high; treatment with one or more therapies selected from a GLP-1 receptor agonist, a weight lowering medication, surgery, radiation therapy, focal ablation, androgen hormone reducing therapy using either androgen deprivation therapy or androgen receptor signaling inhibition therapy as a monotherapy when the risk score is high and the BMI is high; or the treatment comprises treatment with one or more therapies selected from androgen hormone reducing therapy using either androgen deprivation therapy or androgen receptor signaling inhibition therapy or a combination thereof, an PDE4D7 expression increasing therapy, surgery, radiation therapy, chemotherapy, targeted radionuclide therapy, and immunotherapy, when the risk score is high and BMI is low. In a further preferred embodiment the treatment comprises treatment with a GLP-1 receptor agonist supplemented with one or more therapies selected from a weight lowering medication, surgery, focal ablation, androgen hormone reducing therapy using either androgen deprivation therapy or androgen receptor signaling inhibition therapy as a monotherapy when the risk score is high and the BMI is high.

In an embodiment the BMI and the risk score are combined by regression. More specifically, Cox proportional hazards regression can be used to evaluate the effect of the risk score and the BMI, to, for example but not limited to, recurrence free survival, biochemical free survival or overall survival.

Thus the invention describes a method for predicting an outcome for a prostate cancer subject with International Society for Urological Pathology (ISUP) score of 1 or 2, the method comprising: determining expression levels or receiving determined expression levels of PDE4D isoforms PDE4D5, PDE4D7 and PDE4D9 in a sample obtained from the subject; receiving the Cancer of the Prostate Risk Assessment (CAPRA) score determined for the subject; determining a risk score based on the PDE4D5, PDE4D7 and PDE4D9 expression levels and the CAPRA score; wherein the method further comprises receiving or determining the Body Mass Index (BMI) of the subject; wherein the risk score is a high risk score or a low risk score, and the BMI is a high BMI or a low BMI; and proposing a primary treatment for the subject based on the risk score and the BMI. In an embodiment the recommended treatment is: active surveillance with de-escalation of monitoring when the risk score is low and the BMI is low or high; treatment with one or more therapies selected from a GLP-1 receptor agonist, a weight lowering medication, surgery, radiation therapy, focal ablation, androgen hormone reducing therapy using either androgen deprivation therapy or androgen receptor signaling inhibition therapy as a monotherapy when the risk score is high and the BMI is high; or the treatment comprises treatment with one or more therapies selected from androgen hormone reducing therapy using either androgen deprivation therapy or androgen receptor signaling inhibition therapy or a combination thereof, an PDE4D7 expression increasing therapy, surgery, radiation therapy, chemotherapy, targeted radionuclide therapy, and immunotherapy, when the risk score is high and BMI is low.

The invention further relates to a method of treating, preventing or ameliorating a subject with prostate cancer with an ISU score of 1 or 2, the method comprising; determining expression levels or receiving determined expression levels of PDE4D isoforms PDE4D5, PDE4D7 and PDE4D9 in a sample obtained from the subject; receiving the Cancer of the Prostate Risk Assessment (CAPRA) score determined for the subject; determining a risk score based on the PDE4D5, PDE4D7 and PDE4D9 expression levels and the CAPRA score; wherein the method further comprises receiving or determining the Body Mass Index (BMI) of the subject; wherein the risk score is a high risk score or a low risk score, and the BMI is a high BMI or a low BMI; and treating the subject based on the risk score and the BMI. In an embodiment the treatment is: active surveillance with de-escalation of monitoring when the risk score is low and the BMI is low or high; treatment with one or more therapies selected from a GLP-1 receptor agonist, a weight lowering medication, surgery, radiation therapy, focal ablation, androgen hormone reducing therapy using either androgen deprivation therapy or

androgen receptor signaling inhibition therapy as a monotherapy when the risk score is high and the BMI is high; or the treatment comprises treatment with one or more therapies selected from androgen hormone reducing therapy using either androgen deprivation therapy or androgen receptor signaling inhibition therapy or a combination thereof, an PDE4D7 expression increasing therapy, surgery, radiation therapy, chemotherapy, targeted radionuclide therapy, and immunotherapy, when the risk score is high and BMI is low. In an embodiment the treatment comprises treatment with a GLP-1 receptor agonist when the risk score is high and the BMI is high. In a further preferred embodiment the treatment comprises treatment with a GLP-1 receptor agonist supplemented with one or more therapies selected from a weight lowering medication, surgery, radiation therapy, focal ablation, androgen hormone reducing therapy using either androgen deprivation therapy or androgen receptor signaling inhibition therapy as a monotherapy when the risk score is high and the BMI is high. In an embodiment the treatment comprises treatment with androgen hormone reducing therapy using either androgen deprivation therapy or androgen receptor signaling inhibition therapy or a combination thereof and/or an PDE4D7 expression increasing therapy, when the risk score is high and BMI is low. In an embodiment the treatment comprises treatment with androgen hormone reducing therapy using either androgen deprivation therapy or androgen receptor signaling inhibition therapy or a combination thereof and/or an PDE4D7 expression increasing therapy supplemented with one or more therapies selected from surgery, radiation therapy, chemotherapy, targeted radionuclide therapy, and immunotherapy, when the risk score is high and BMI is low.

When used herein the term active surveillance refers to the active monitoring of a tumor, for example by monitoring for changes in tumor size, shape or structure, or in the case of radical prostatectomy, monitoring signs of a new tumor arising, for example by monitoring plasma PSA levels. The term implies that no active anti-tumor therapy is conducted, such as drugs, radiation therapy or surgery. The term active surveillance may refer to the situation post-surgery, meaning that surgery is performed and followed up with active surveillance (close monitoring but no further treatment). In this context, active surveillance with de-escalation of monitoring implies that the intensity of monitoring is reduced over time.

GLP-1 receptor agonist, also known as Glucagon-like peptide-1 (GLP-1) receptor agonists, GLP-1 analogs, GLP-1Ras, GLP-1DAs or incretin mimetics, are a class of anorectic drugs that reduce blood sugar and energy intake by activating the GLP-1 receptor. Such compound may be used in the treatment of diabetes and/or obesity. When used herein the term may be used to refer to any compound which when administered to a patient activates GLP-1 receptor activity and results in loss of weight. Non limiting exemplary GLP-1 receptor agonists are dulaglutide, exenatide, exenatide extended-release, liraglutide, lixisenatide, semagluride injection, emaglutide tablets, albiglutide, and tirzepatide.

When used herein the term "weight lowering medication" intends to any drug or compound resulting in weight loss, preferably resulting in a lower BMI. Thus the term also includes GLP-1 receptor agonists as referred herein. In addition, the term may refer to non GLP-1 receptor agonst drugs, such as but not limited to phentermine,orlistat, lorcaserin, phendimetrazine, methamphetamine, benzphetamine, diethylpropion, sibutramine, phentermine and topiramate extended-release, phentermine, orlistat oral, and bupropion and naltrexone.

When used herein the term surgery may refer to radical prostatectomy, partial removal of the prostate, orchiectomy, or removal of a metastasis, or a combination of two or more of these. Preferably the term refers to radical prostatectomy and/or orchiectomy, most preferably to radical prostatectomy.

When used herein the term radiation therapy intends to refer to treatment of the primary tumor (prostate tumor) or a metastasis with radiation. The therapy may include but is not limited to external beam radiation, brachytherapy (internal radiation therapy) and radiopharmaceuticals. Several forms of external beam radiation therapy are known, such as Three-dimensional conformal radiation therapy (3D-CRT), Intensity modulated radiation therapy (IMRT), Stereotactic body radiation therapy (SBRT), MRI-guided radiation therapy, and Proton beam radiation therapy. The brachytherapy may be Permanent (low dose rate, or LDR) brachytherapy or Temporary (high-dose rate, or HDR) brachytherapy. The radiopharmaceuticals may be radiopharmaceuticals that target Prostate-specific membrane antigen (PSMA), which is a protein that is often found in large amounts on prostate cancer cells. Non limiting examples are Lutetium Lu-177 vipivotide tetraxetan (also known as 177Lu-PSMA-617 or Pluvicto). Alternatively the radiopharmaceuticals may also be radiopharmaceuticals that target the bones, such as but not limited to Radium-223 (Xofigo), Strontium-89 (Metastron), or Samarium-153 (Quadramet).

When used herein the term focal ablation therapy intends to refer to types of treatment for localized prostate cancer that target only the area of the prostate where the tumor is located. The method is suitable for tumors which are confined to the prostate, preferably only on one side, without spreading to other tissue or organs around the prostate. Each type of focal therapy uses an energy source (like heat, cold or electric shock) to destroy tumor cells. Non limiting examples are Cryotherapy, Focal laser ablation (FLA), High-intensity focused ultrasound (HIFU), Irreversible electroporation (IRE), and Transurethral ultrasound ablation of the prostate (TULSA).

When used herein the term androgen hormone reducing therapy refers a therapy that reduces androgen receptor signaling in the tumor environment. This may be achieved for example by blocking androgen signaling, or reducing available androgen. For example the androgen hormone reducing therapy may be androgen deprivation therapy (ADT) or androgen receptor signaling inhibition therapy (ARSI therapy), or a combination thereof. The term androgen hormone

reducing monotherapy implies that either ADT or ARSI therapy is used, not both.

When used herein androgen deprivation therapy (ADT), also referred to as androgen suppression therapy, refers to an antihormone therapy. Androgen deprivation therapy aims to lower the levels of androgen hormones (such as testosterone) in the patient, as prostate cancer cells are usually dependent on androgen hormones for growth. ADT may comprise surgery based methods or drug based methods. For example orchiectomy (surgical removal of the testicles) may be used as ADT, as the testicles are main organ where androgens are produced. Alternatively a drug based method can be used, some non-limiting examples being chemical castration and antiandrogen treatment. Chemical castration may for example be achieved by agonists or antagonists of the gonadotropin releasing hormone (GnRH). GnRH induces Luteinizing Hormone production which in turn induces testosterone synthesis. GnRH agonists and antagonists used in androgen deprivation therapy include leuprorelin (leuprolide), goserelin, triptorelin, histrelin, buserelin, and degarelix. Antiandrogens treatment aims to reduce Androgen Receptor (AR) signaling induced androgen synthesis by blocking the AR signaling pathway, for example by disrupting the positive feedback loop created by testosterone, or alternatively target testosterone synthesis or AR nuclear translocation.

Thus the ADT may consist or comprises one or more of androgen receptor antagonsits, androgen synthesis antagonists, or antigonadotropins. When used herein the term androgen receptor antagonist may refer to a steroidal antiandrogen or a non-steroidal antiandrogen. Non limiting examples of steroidal antiandrogens are: 17$\alpha$-Hydroxyprogesterone derivatives such as chlormadinone acetate, cyproterone acetate, megestrol acetate, or osaterone acetate; 19-Norprogesterone derivative such as nomegestrol acetate; 19-Nortestosterone derivatives such as dienogest or oxendolone, 17$\alpha$-Spirolactone derivatives such as drospirenone or spironolactone; or other drugs not belonging to the above categories such as medrogestone.

Non limiting examples of nonsteroidal antiandrogens are bicalutamide, flutamide, nilutamide, apalutamide, darolutamide, enzalutamide, proxalutamide, cimetidine, and topilutamide.

Non limiting examples of androgen synthesis inhibitors are CYP17A1 inhibitors such as abiraterone acetate, ketoconazole and seviteronel; CYP11A1 (P450scc) inhibitors such as aminoglutethimide; 5$\alpha$-Reductase inhibitors such alfatradiol, dutasteride, epristeride, finasteride, and saw palmetto extract.

Non limiting examples of antigonadotropins are Estrogens such as estradiol (and its esters), ethinylestradiol, conjugated estrogens, and diethylstilbestrol; CmRH analogues such as GnRH agonists (e.g., goserelin, leuprorelin) or GnRH antagonists (e.g., cetrorelix); rogestogens such as chlormadinone acetate, cyproterone acetate, gestonorone caproate, medroxyprogesterone acetate, and megestrol acetate.

Some exemplary examples of antiandrogens that may be used as ADT are cyproterone acetate, flutamide, nilutamide, bicalutamide, enzalutamide, abiraterone, abiraterone acetate, seviteronel, apalutamide, darolutamide, Leuprorelin and galeterone. Thus in an embodiment, the androgen deprivation therapy comprises treatment with an antiandrogen. In an embodiment the antiandrogen is selected from cyproterone acetate, flutamide, nilutamide, bicalutamide, enzalutamide, abiraterone, abiraterone acetate, seviteronel, apalutamide, darolutamide, Leuprorelin and galeterone, more preferably selected from enzalutamide, Leuprorelin, abiraterone, or apalutamide.

When used herein the term Neoadjuvant androgen deprivation therapy (NADT) refers to systemic therapy administered after the diagnosis of prostate cancer but before locoregional therapy such as radical prostatectomy (RP) or radiation. With NADT prior to RP, the intent is to eradicate malignant androgen-dependent cells, in the hope that sufficient tumor regression will permit complete resection of residual prostate cancer, improving pathologic outcome and survival. Therefore according to the invention the androgen deprivation therapy is neoadjuvant androgen deprivation therapy. The examples of androgen deprivation therapy listed may also be use as neoadjuvant androgen deprivation therapy. When used herein the broader term ADT is intended to also include NADT.

When used herein the term androgen receptor signaling inhibition (ARSI) therapy specifically refers to a medicament that inhibits androgen receptor signaling. Thus ARSI therapy is a specific form of ADT. The androgen receptor agonist may be a steroidal antiandrogen (SAA) or a non-steroidal antiandrogen (NSAA). Non limiting examples of steroidal antiandrogens are: 17$\alpha$-Hydroxyprogesterone derivatives such as chlormadinone acetate, cyproterone acetate, megestrol acetate, or osaterone acetate; 19-Norprogesterone derivative such as nomegestrol acetate; 19-Nortestosterone derivatives such as dienogest or oxendolone, 17$\alpha$-Spirolactone derivatives such as drospirenone or spironolactone; or other drugs not belonging to the above categories such as medrogestone.

Non limiting examples of nonsteroidal antiandrogens are bicalutamide, flutamide, nilutamide, apalutamide, darolutamide, enzalutamide, proxalutamide, cimetidine, and topilutamide.

Non limiting examples of androgen synthesis inhibitors are CYP17A1 inhibitors such as abiraterone acetate, ketoconazole and seviteronel; CYP11A1 (P450scc) inhibitors such as aminoglutethimide; 5$\alpha$-Reductase inhibitors such alfatradiol, dutasteride, epristeride, finasteride, and saw palmetto extract.

When used herein the term PDE4D7 expression increasing therapy refers to a compound or biological that increase expression of PDE4D7 or increases activity of the PDE4D7 enzyme. the inventors found that re-expressing PDE4D7 in these knockdown cells reduced proliferation. Furthermore expression of PDE4D7 was able to reduce resistance to drug treatments such as antiandrogen resistance, PARP inhibitor resistance or chemotherapy resistance.

Based on these data the inventors concluded that increasing expression or increasing PDE4D7 phosphodiesterase activity reduces therapy resistance and may be used to treat therapy resistant prostate cancers.

Therefore in a first aspect the invention relates to a product for use in the treatment, prevention or amelioration of prostate cancer by reducing therapy resistance in a subject in need thereof, wherein the product induces the expression of PDE4D7 or promotes activity of phosphodiesterase 4D7. Alternatively the invention relates to a method of treating, preventing or ameliorating prostate cancer in a subject in need thereof, the method comprising administering to the subject product induces the expression of PDE4D7 or promotes activity of phosphodiesterase 4D7 and is selected from:

a polynucleotide encoding a peptide having a sequence according to SEQ ID NO: 20 or a variant thereof, wherein the variant is a polynucleotide encoding a peptide having a sequence with at least 90% sequence homology with SEQ ID NO: 20 and wherein the peptide has phosphodiesterase activity; or
a polynucleotide comprising a nucleotide sequence as defined in SEQ ID NO: 19 or a variant thereof, wherein the variant is a polynucleotide comprising a nucleotide sequence with at least 90% sequence homology to SEQ ID NO: 19 and wherein the polynucleotide encodes a peptide that has phosphodiesterase activity; or
a peptide comprising a peptide sequence as defined in SEQ ID NO: 20 or a variant thereof, wherein the variant is a peptide comprising a peptide sequence with at least 90% sequence homology with SEQ ID NO: 20 and wherein the peptide has phosphodiesterase activity; or
a peptide comprising a peptide sequence encoded by a polynucleotide comprising a nucleotide sequence as defined in SEQ ID NO: 19, or a variant thereof, wherein the variant is peptide comprising a peptide sequence encoded by a polynucleotide comprising a nucleotide sequence with at least 90% sequence homology to SEQ ID NO: 19 and wherein the polynucleotide encodes a peptide that has phosphodiesterase activity; or
a compound as defined in formula I

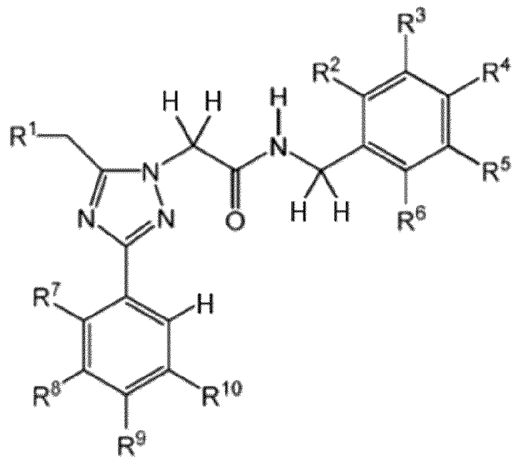

Formula I

wherein

$R_1$ is H, (C1-4)alkyl or (C1-4)alkyloxy;
$R_2$ and $R_6$ are independently selected from Hand;
$R_3$, $R_4$ and $R_5$ are independently selected from H, halogen, CN, $(C_{1-4})$alkyl and $(C_{1-4})$alkyloxy, the $(C_{1-4})$alkyl and $(C_{1-4})$alkyloxy groups being optionally substituted with 1 to 3 fluoros;
$R_7$, $R_8$, and $R_{10}$ are independently selected from Hand F;
$R_9$ is selected from (C1-4)alkyl, (C1-4)alkyloxy, CN and halogen, the (C1-4)alkyl and (C1-4)alkyloxy groups being optionally substituted with 1 to 3 fluoros;
or
a peptide having a sequence comprising or consisting a sequence selected from SEQ ID Nos: 24, 25, 26 or 27, or a polynucleotide encoding a peptide having a sequence comprising or consisting a sequence selected from SEQ ID Nos: 74, 75, 76, or 77.

When used herein, a gene therapy for inducing PDE4D7 expression refers to a method to introduce a nucleotide encoding the phosphodiesterase 4D7 isoform in a cell, preferably a cell of a subject having prostate cancer, more preferably in a prostate cancer cell or a metastasis thereof. The nucleotide may for example be a viral vector or a non-viral

vector, however it is understood that any nucleotide which can be introduced in a cell of a patient and express PDE4D7 may be used. For example the nucleotide may also be an mRNA encoding PDE4D7 or a variant of PDE4D7. Nucleotides such as mRNA may be expressed in a tumor cell in vivo using lipid nanoparticles (LNP). Suitable LNP formulations are known to the skilled person.

Viral vectors are a known strategy for gene therapy, typically viral-vector gene therapies use modified viruses as drug-delivery vehicles to introduce specific DNA or RNA sequences into cells. The vector is packaged using the viral proteins allowing infection of cells and expression of its viral genome. Typically, the viral vector is modified such that no new viral particles can be produced upon infection of the target cell. Non limiting examples include retroviruses (RV), adenoviruses (AV), adeno-associated viruses (AAV), lentiviruses (LV), and herpes simplex viruses (HSV). Other ways to use viral particles or viral vectors to introduce the PDE4D7 encoding nucleotide are known to the person skilled in the art and also envisaged to be encompassed by the invention.

Alternatively, to viral vector based gene therapy, a non-viral vector may be used. These vectors typically contain a promotor to drive expression of the relevant construct (e.g. PDE4D7), and typically require some means to introduce the vector into the target cell. Means to deliver a non-viral vector to a target cell in a subject are known to the skilled person, and for example reviewed in Ramamoorth M, Narvekar A. Non viral vectors in gene therapy- an overview. J Clin Diagn Res. 2015 Jan;9(1):GE01-6 (hereby incorporated by reference in its entirety). For example, nanoparticles can be used to encapsulate the vector. Non limiting examples are lipid based nanoparticles, peptide based nanoparticles, cationic lipid based nanoparticles, apolipoprotein based nanoparticles, (synthetic) polymer based nanoparticles such as polyethylenimine (PEI), chitosan, polylactate, polylactide, polyglucoside, denriomer or polymethracylate based nanoparticles. When used herein a nanoparticle is a small particle which can be used a sa carrier to deliver a cargo (payload) in a patient. Preferably the cargo is the product as broadly defined herein. Therefore a nanoparticle can be used to deliver the product induces the expression of PDE4D7 or promotes activity of phosphodiesterase 4D7 to a site in patient, e.g. a cell, tissue or organ. Other ways to use non- viral vectors to introduce the PDE4D7 encoding nucleotide are known to the person skilled in the art and also envisaged to be encompassed by the invention.

Therefore, in an embodiment the gene therapy is selected from: a viral vector capable of expressing PDE4D7 in a subject or a non-viral vector capable of expressing PDE4D7 in a subject. In an embodiment the product is delivered using a nanoparticle.

When used herein the term vector is used to indicate any particle (e.g., a plasmid, a cosmid, a Lambda phage) used as a vehicle to artificially carry a foreign nucleic sequence - usually DNA - into another cell, where it can be replicated and/or expressed.

When used herein a compound that induces PDE4D7 expression is intended to refer to any biological or chemical compound that is able to increase the expression of PDE4D7. This may for example be achieved by promoting transcription of the PDE4D7 gene or inhibiting degradation of the phosphodiesterase 4D7 isoform protein. For example the compound may engage a signal transduction pathway to indirectly promote transcription of the PDE4D7 or directly interact with the promoter region of the genomic DNA to promote transcription of the PDE4D7 isoform.

When used herein promoting transcription of PDE4D7 refers to increasing the transcription of PDE4D7 resulting in a higher amount of PDE4D7 mRNA transcripts, and/or preferably in a higher amount of phosphodiesterase 4D7 isoform protein in the cell. The promoting may be specific for PDE4D7, specific for all PDE4D isoforms, specific for all PDE4 or even all PDE family members. In other words, increasing expression of PDE4D7 does not exclude that other PDE4D isoforms, other PDE4 family members or even other PDE family members are also increased in expression.

When used herein, a compound directly stimulating or promoting phosphodiesterase 4D7 activity is intended to refer to a compound which directly engages with the phosphodiesterase 4D7 isoform protein enzymatic activity. Without wishing to be bound be theory, it is theorized that PDE proteins such as the PDE4D7 protein can exist in an active and inactive conformation, wherein activity can be induced by other compounds through interaction with the PDE protein, for example by promoting an active conformation of the protein or by blocking or preventing binding of inhibitors, or by modifying the protein to promote activity (for example by phosphorylation or other known protein modifications). When used herein, enzymatic activity when referring to a phosphodiesterase refers to catalysis of the hydrolysis of cAMP and/or cGMP.

When used herein, a compound indirectly stimulating or promoting phosphodiesterase 4D7 activity is intended to refer to a compound which indirectly engages with the phosphodiesterase 4D7 isoform protein enzymatic activity. Similarly as above it is envisaged that compounds may induce activators of PDE4D7 or block inhibitors form PDE4D7 activity and thus indirectly affect the protein's enzymatic activity. The person skilled in the art is aware of methods to determine PDE or specifically PDE4D7 activity. For example, commercial products are available to assay for PDE activity, and methods have for example been described in Blair et al. Measuring cAMP Specific Phosphodiesterase Activity: A Two-step Radioassay. Bio Protoc. 2020 Apr 5;10(7):e3581, hereby incorporated in its entirety by reference.

When used herein, an inhibitor of a transcriptional inhibitor of PDE4D7 refers to a compound capable of blocking an inhibitor of PDE4D7 gene transcription. The inbitior of PDE4D7 gene transcription may be a direct inhibitor capable of binding the genomic DNA and preventing or reducing gene transcription or an indirect inhibitor which through downstream

actions reduces or inhibits transcription of the PDE4D7 gene.

When used herein an inhibitor of an inhibitor of phosphodiesterase 4D7 enzymatic activity refers to a compound that can block an inhibitor of phosphodiesterase isoform 4D7 protein activity. For example the compound may function by degrading the inhibitor, preventing the inhibitor from engaging with PDE4D7 or by inhibiting the activity of the inhibitor.

When used herein, mRNA encoding a phosphodiesterase 4D7 protein refers to a RNA molecule from which the protein corresponding to SEQ ID NO: 20 or a substantially similar protein can be translated. The mRNA molecule generally comprises non translated elements such as 5' and 3' UTRs. It is anticipated that by direct introduction of PDE4D7 mRNA into the target cell (e.g. a prostate cancer cell in a subject) PDE4D7 can by upregulated. Method of delivering mRNA to a target cell are known to the skilled person, for example using nanobodies as described above.

When used herein a phosphodiesterase 4D7 protein refers to a compound comprising PDE4D7 protein or a biosimilar, or a constitutively active variant thereof, having phosphodiesterase activity. It is anticipated that by direct introduction of PDE4D7 protein into the target cell (e.g. a prostate cancer cell in a subject) PDE4D7 activity can be increased. Method of delivering protein to a target cell are known to the skilled person, for example using nanobodies as described above.

When used herein, a phosphodiesterase 4D7 activity promoting peptide refers to a peptide that is able to increase activity of the PDE4D7 enzyme. Non limiting examples are Amyloid beta (Abeta) peptides, which have been demonstrated to specifically increase long PDE4D isoforms such as PDE4D7. When used herein Amyloid beta, also known as Aβ or Abeta, refers to peptides of 37-49 amino acids (Chen et al. Acta Pharmacol Sin 38, 1205-1235 (2017)) that are the main component of the amyloid plaques found in the brains of people with Alzheimer's disease. Amyloid beta peptide (Aβ) is produced through the proteolytic processing of a transmembrane protein, amyloid precursor protein (APP), by β- and γ-secretases.

Therefore, the phosphodiesterase 4D7 activity promoting peptide is preferably a Abeta peptide or derivative thereof. When used herein a Abeta peptide or derivative thereof is characterized as a peptide having a length of 16 to 50 amino acids and comprising an amino acid sequence 90%, 91%, 92%, 93%, 94%, 95%, 96%, 97%, 98%, 99% or 100% identical to the sequence defined by SEQ ID NO: 74 below (Abeta core peptide 1), more preferably comprising an amino acid sequence 90%, 91%, 92%, 93%, 94%, 95%, 96%, 97%, 98%, 99% or 100% identical to the sequence defined by SEQ ID NO: 75 below (Abeta core peptide 2). Non limiting examples are provided by Abeta 42 (SEQ ID NO: 76) and Abeta 40 (SEQ ID NO: 77). Therefore in an embodiment the Abeta peptide or derivative thereof is characterized as a peptide having a length of 42 to 50 amino acids and comprising an amino acid sequence 90%, 91%, 92%, 93%, 94%, 95%, 96%, 97%, 98%, 99% or 100% identical to the sequence defined by SEQ ID NO: 76, or a peptide having a length of 40 to 50 amino acids and comprising an amino acid sequence 90%, 91%, 92%, 93%, 94%, 95%, 96%, 97%, 98%, 99% or 100% identical to the sequence defined by SEQ ID NO: 77.

SEQ ID NO: 74 Abeta core peptide 1
HDSGYEVHHQKLVFFAEDVGSNKGAIIG
SEQ ID NO: 75 Abeta core peptide 2
FRHDSGYEVHHQKLVFFAEDVGSNKGAIIGLMV
SEQ ID NO: 76 Abeta 42
DAEFRHDSGYEVHHQKLVFFAEDVGSNKGAIIGLMVGGVVIA
SEQ ID NO: 77 Abeta 40
DAEFRHDSGYEVHHQKLVFFAEDVGSNKGAIIGLMVGGVV

When used herein the term chemotherapy refers to a chemotherapeutic agents, also known as cytotoxic agents or cytostatic drugs, that are known to be of use in chemotherapy for cancer, and have the intention to kill tumor cells or reduce tumor size. Non limiting examples are Alkylating agents such as Altretamine, Bendamustine, Busulfan, Carboquone, Carmustine, Chlorambucil, Chlormethine, Chlorozotocin, Cyclophosphamide, Dacarbazine, Fotemustine, Ifosfamide, Lomustine, Melphalan, Melphalan flufenamide, Mitobronitol, Nimustine, Nitrosoureas, Pipobroman, Ranimustine, Semustine, Streptozotocin, Temozolomide, Thiotepa, Treosulfan, Triaziquone, Triethylenemelamine, Trofosfamide, and Uramustine; or Anthracyclines such as Aclarubicin, Daunorubicin, Doxorubicin, Epirubicin, Idarubicin, Mitoxantrone, Pirarubicin, Valrubicin, and Zorubicin; or Cytoskeletal disruptors (taxanes) such as Abraxane, Cabazitaxel, Docetaxel, Larotaxel, Paclitaxel, Taxotere, and Tesetaxel; or Epothilones such as Ixabepilone; or Histone deacetylase inhibitors such as Entinostat, Romidepsin, Vorinostat, and Zabadinostat; or Inhibitors of topoisomerase I such as Belotecan, Camptothecin, Exatecan, Gimatecan, Irinotecan, and Topotecan; or Inhibitors of topoisomerase II such as Etoposide, Teniposide, and Tafluposide; or Kinase inhibitors such as Bortezomib, Erlotinib, Gefitinib, Imatinib, Vemurafenib, and Vismodegib; or Nucleotide analogs and precursor analogs such as Azacitidine, Azathioprine, Capecitabine, Cladribine, Clofarabine, Cytarabine, Decitabine, Doxifluridine, Fludarabine, Fluorouracil, Gemcitabine, Hydroxyurea, Mercaptopurine, Methotrexate, Nelarabine, Pemetrexed, and Tioguanine (formerly Thioguanine); or Peptide antibiotics such as Actinomycin, and Bleomycin; or Platinum-based agents such as Main Carboplatin, Cisplatin, Dicycloplatin, Oxaliplatin,

Nedaplatin, and Satraplatin; or Retinoids such as Alitretinoin, Bexarotene, and Tretinoin; or Vinca alkaloids and derivatives such as Vinblastine, Vincristine, Vindesine, and Vinorelbine.

When used herein targeted radionuclide therapy refers to radioisotopes bound to or incorporated in a targeting agent such as PSMA or an antibody such as a nanobody. A non-limiting example suitable for prostate cancer is PSMA Lutetium-177.

Non limiting examples of immunotherapy are cancer vaccine (such as but not limited to Sipuleucel-T (Provenge) and prostatic acid phosphatase (PAP)), or Immune checkpoint inhibitors, such as but not limited to Ipilimumab, Nivolumab, Pembrolizumab, Cemiplimab,

Spartalizumab, Camrelizumab, Sintilimab, Tislelizumab, Toripalimab, Dostarlimab, Retifanlimab, AMP-224, MEDI0680, Atezolizumab, Avelumab, Durvalumab, Envafolimab,

Cosibelimab, AUNP-12, CA-170, anti-OX40 antibody BMS 986178, Tremelimumab, Abatacept, Belatacept, Sotorasib, Adagrasib, Relatlimab, Lisavanbulin, PHI-101, and Quemliclustat.

In a second aspect the invention relates to a computer program product comprising instructions which, when the program is executed by a computer, cause the computer to carry out a method comprising:
determining a risk score based on received PDE4D5, PDE4D7 and PDE4D9 expression levels and a received CAPRA score, the expression levels being determined in a sample of a prostate cancer subject with ISUP score of 1 or 2, and the CAPRA score obtained from the subject; determining an outcome for the subject based on the risk score and a received BMI from the subject; wherein the outcome is a favorable outcome or a non-favorable outcome.

In a third aspect the invention relates to the use of a kit to determine an outcome for a prostate cancer subject with ISUP score of 1 or 2, the use comprising using the kit to determine the expression levels of PDE4D isoforms PDE4D5, PDE4D7 and PDE4D9 in sample obtained from the subject; determining a risk score based on the PDE4D5, PDE4D7 and PDE4D9 expression levels and a CAPRA score obtained from the subject; determining an outcome for the subject based on the risk score and a BMI obtained form the subject; wherein the kit comprises at least one primer and/or probe for determining the gene expression profile for each of PDE4D5, PDE4D7 and PDE4D9, and optionally, at least one primer and/or probe for determining the gene expression profile for one or more reference genes selected from the group consisting of: hypoxanthine phosphoribosyl transferase 1 (HPRT1), Tubulin-Alpha- lb (TUBA1B) pumilio RNA-Binding Family Member (PUM1), and TATA box binding protein (TBP); and optionally, at least one agent for determining a prostate-specific antigen (PSA) level in a biological sample obtained from the subject.

Thus further disclosed herein is a comprises at least one primer and/or probe for determining the gene expression profile for each of PDE4D5, PDE4D7 and PDE4D9, and optionally, at least one primer and/or probe for determining the gene expression profile for one or more reference genes selected from the group consisting of: hypoxanthine phosphoribosyl transferase 1 (HPRT1), Tubulin-Alpha- lb (TUBA1B) pumilio RNA-Binding Family Member (PUM1), and TATA box binding protein (TBP); and optionally, at least one agent for determining a prostate-specific antigen (PSA) level in a biological sample obtained from the subject.

Depending on the type of primers and or probes the expression levels of PDE4D isoforms PDE4D5, PDE4D7 and PDE4D9 can be determined using the kit with techniques commonly known within the field. For example the primers and optionally probes may be suitable for a PCR based technique such as qPCR, or primers for directed mRNA sequencing.

In a fourth aspect the invention relates to a GLP-1 receptor agonist for use in the treatment of prostate cancer in a subject with ISUP score 1 or 2. Alternatively the invention describes a method of treating prostate cancer in a subject in need thereof, wherein the subject has prostate cancer with ISUP score 1 or 2 and the treatment comprises administering a GLP-1 receptor agonist. In an embodiment the a GLP-1 receptor agonist is administered if the BMI of the subject is high. In an emboimdnet the BMI is 25 or higher, preferably 26, 27, 28 or even 29 or higher.

In an embodiment the invention describes a GLP-1 receptor agonist for use in the treatment of prostate cancer in a subject with ISUP score 1 or 2, wherein the use comprises: determining or receiving the determined expression levels of PDE4D isoforms PDE4D5, PDE4D7 and PDE4D9 in a sample obtained from the subject; receiving the CAPRA score determined for the subject; determining a risk score based on the PDE4D5, PDE4D7 and PDE4D9 expression levels and the Cancer of the Prostate Risk Assessment (CAPRA) score; receiving or determining the Body Mass Index (BMI) of the subject; wherein the GLP-1 receptor agonist is administered to the subject when the risk score is high and the BMI is high. Alternatively the invention describes a method of treating prostate cancer in a subject in need thereof, wherein the subject has prostate cancer with ISUP score 1 or 2 and the treatment comprises determining or receiving the determined expression levels of PDE4D isoforms PDE4D5, PDE4D7 and PDE4D9 in a sample obtained from the subject; receiving the CAPRA score determined for the subject; determining a risk score based on the PDE4D5, PDE4D7 and PDE4D9 expression levels and the Cancer of the Prostate Risk Assessment (CAPRA) score; receiving or determining the Body Mass Index (BMI) of the subject; wherein the GLP-1 receptor agonist is administered to the subject when the risk score is high and the BMI is high.

In an embodiment the GLP-1 receptor agonist is selected from dulaglutide, exenatide, exenatide extended-release, liraglutide, lixisenatide, semagluride injection, emaglutide tablets, albiglutide, and tirzepatide.

EXAMPLES

For the examples the following patient cohort is used as also described in detail in Van Strijp et al., Prostate Cancer, Volume 2018, Issue 1, Article ID 5821616 (hereby incorporated by reference in its entirety):
RP patient cohort (n=550): patients consecutively managed at a single, large-volume prostate cancer center were included into the study (Martini-Klinik, Hamburg, Germany). Two small biopsy punches (~1×2 mm) of a representative resected tumor area of patients operated on between 2000 and 2004 were collected from the tumors index lesion. Patients who underwent adjuvant hormone therapy were removed from the study cohort. RP* patient cohort (n=130): detailed characteristics of this cohort and analysis of the respective gene expression data were described previously [Taylor et al., Cancer Cell. (2010) 18, no. 1, 11-22, hereby incorporated by reference in its entirety]. DB patient cohort (n=168): from the tumor positive diagnostic biopsy with the highest Gleason grade per patient a single biopsy punch (~1×2 mm) was collected. Patients were diagnosed with prostate cancer and operated on between 1994 and 2011 at the Prostate Center (University Hospital Münster, Germany). After quality control of the study data based on predefined criteria 503 and 151 patient samples were defined eligible for statistical analysis in the RP and the DB cohort, respectively. From these patients were selected with ISUP score 1 (75 patients) or ISUP score 1 or 2 (112 patients), of these groups BMI information was available for 70 patients with ISUP 1 and 104 patients with ISUP 1 or 2.

*Reference Example 7 - Method for stratifying prostate cancer patients based on the Risk Score only*

This method describes an exemplary method for prognosis for prostate cancer based on previously published Risk Score (combination of CAPRA and PDE4D5, PDE4D7, and PDE4D9 expression levels):

*Development of the model*

In a first step, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.
In a next step, gene expression profiles for DE4D5, PDE4D7, and PDE4D9 are obtained for each of the biological samples obtained from the first set of patients, e.g., by performing RT-qPCR (real-time quantitative PCR) on RNA extracted from each biological sample. The exemplary gene expression profiles include expression levels (e.g., values) for DE4D5, PDE4D7, and PDE4D9 which can be normalized using values for each of a set of reference genes, such as HPRT1, TUBA1B, PUM1, and/or TBP. In one realization, the gene expression profile values of DE4D5, PDE4D7, and PDE4D9 are normalized to with respect to one or more reference genes selected from the group consisting of HPRT1, TUBA1B, PUM1, and TBP, e.g., at least one, or at least two, or at least three, or, preferably, all of these reference genes.
In a next step, a scoring function for assigning an expression based risk score is determined, based on the gene expression profile for DE4D5, PDE4D7, and PDE4D9 obtained for at least some of the biological samples obtained for the first set of patients and respective results obtained from the monitoring. In one preferred realization, the scoring function is a linear transformation that transforms the normalized gene expression profile into a predefined range of values, such as the above-mentioned range of 1 to 5. As mentioned above, such a transformation can be determined by considering the frequency distribution of the normalized gene expression profile values for DE4D5, PDE4D7, and PDE4D9 for biological samples of a population of prostate cancer subjects (here, the first set of patients) and by determining the transformation that transforms the frequency distribution into the desired range. In one particular realization, the expression based risk score is determined as specified above.

*Implementation of the model*

In a first step, a biological sample is obtained from a patient (subject or individual). The patient can be a new patient or one of the first set.
In a next step, a gene expression profile is obtained for DE4D5, PDE4D7, and PDE4D9, e.g., by performing PCR or RNA sequencing on the biological sample. In one realization, the gene expression profile values of DE4D5, PDE4D7, and PDE4D9 are normalized to with respect to one or more reference genes selected from the group consisting of HPRT1, TUBA1B, PUM1, and TBP, e.g., at least one, or at least two, or at least three, or, preferably, all of these reference genes. Other reference genes which may be additionally or alternatively used in this step or the corresponding step in developing the model include: actin, beta, mRNA (ACTB); 60S acidic ribosomal phosphoprotein P0 mRNA (RPLPO); Polymerase (RNA) II (DNA Directed) Polypeptide A, 220kDa (POLR2A); Beta-2-Microglobulin (B2M); and Aminolevulinate-Delta-Synthase (ALAS-1).
In a next step, an expression based risk score is determined for the patient, based on the gene expression profile, using the derived scoring function. In a next step, a risk score is determined for the patient based on the expression based

risk score and the CAPRA score of the patient. This has been described in more detail above.

*Example 2 - Method for stratifying prostate cancer patients based on the Risk Score and BMI*

This method describes a method for prognosis for prostate cancer based on previously published Risk Score (combination of CAPRA and PDE4D5, PDE4D7, and PDE4D9 expression levels) combined with BMI:

*Development of the model*

In a first step, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.

In a next step, gene expression profiles for DE4D5, PDE4D7, and PDE4D9 are obtained for each of the biological samples obtained from the first set of patients, e.g., by performing RT-qPCR (real-time quantitative PCR) on RNA extracted from each biological sample. The exemplary gene expression profiles include expression levels (e.g., values) for DE4D5, PDE4D7, and PDE4D9 which can be normalized using values for each of a set of reference genes, such as HPRT1, TUBA1B, PUM1, and/or TBP. In one realization, the gene expression profile values of DE4D5, PDE4D7, and PDE4D9 are normalized to with respect to one or more reference genes selected from the group consisting of HPRT1, TUBA1B, PUM1, and TBP, e.g., at least one, or at least two, or at least three, or, preferably, all of these reference genes.

In a next step, a scoring function for assigning an expression based risk score is determined, based on the gene expression profile for DE4D5, PDE4D7, and PDE4D9 obtained for at least some of the biological samples obtained for the first set of patients and respective results obtained from the monitoring. In one preferred realization, the scoring function is a linear transformation that transforms the normalized gene expression profile into a predefined range of values, such as the above-mentioned range of 1 to 5. As mentioned above, such a transformation can be determined by considering the frequency distribution of the normalized gene expression profile values for DE4D5, PDE4D7, and PDE4D9 for biological samples of a population of prostate cancer subjects (here, the first set of patients) and by determining the transformation that transforms the frequency distribution into the desired range. In one particular realization, the expression based risk score is determined as specified above.

*Implementation of the model*

In a first step, a biological sample is obtained from a patient (subject or individual). The patient can be a new patient or one of the first set.

In a next step, a gene expression profile is obtained for DE4D5, PDE4D7, and PDE4D9, e.g., by performing PCR or RNA sequencing on the biological sample. In one realization, the gene expression profile values of DE4D5, PDE4D7, and PDE4D9 are normalized to with respect to one or more reference genes selected from the group consisting of HPRT1, TUBA1B, PUM1, and TBP, e.g., at least one, or at least two, or at least three, or, preferably, all of these reference genes. Other reference genes which may be additionally or alternatively used in this step or the corresponding step in developing the model include: actin, beta, mRNA (ACTB); 60S acidic ribosomal phosphoprotein P0 mRNA (RPLPO); Polymerase (RNA) II (DNA Directed) Polypeptide A, 220kDa (POLR2A); Beta-2-Microglobulin (B2M); and Aminolevulinate-Delta-Synthase (ALAS-1).

In a next step, an expression based risk score is determined for the patient, based on the gene expression profile, using the derived scoring function. In a next step, a risk score is determined for the patient based on the expression based risk score and the CAPRA score of the patient. This has been described in more detail above.

In a next step the BMI of the patient is determined or provided.

In an optional next step, a therapy recommendation may be provided, e.g., to the patient or his or her guardian, to a doctor, or to another healthcare worker, based on the risk score. and the BMI. To this end, the risk score may be categorized into one of a predefined set of risk groups, based on the value of the risk score. In addition, the BMI may be categorized in a predefined risk group. Providing a therapy recommendation may include one or more of: a) proposing a therapy for the patient based on the assigned risk group, with at least two of the risk groups being associated with different therapies, b) computing a disease progression risk prediction of the patient before or after prostate surgery; and c) computing a therapy response prediction for the patient before or after prostate surgery. Example therapies include at least active surveillance with de-escalation of monitoring when the risk score is low and the BMI is low or high; treatment with one or more therapies selected from a GLP-1 receptor agonist, a weight lowering medication, surgery, radiation therapy, focal ablation, androgen hormone reducing therapy using either androgen deprivation therapy or androgen receptor signaling inhibition therapy as a monotherapy when the risk score is high and the BMI is high; or the treatment comprises treatment with one or more therapies selected from androgen hormone reducing therapy using either androgen deprivation therapy or androgen receptor signaling inhibition therapy or a combination thereof, an PDE4D7 expression increasing therapy,

surgery, radiation therapy, chemotherapy, targeted radionuclide therapy, and immunotherapy, when the risk score is high and BMI is low.

*Example 3 - analyzing patient data*

To explore the prognostic power of the Risk Score combined with BMI, the benefit of a combination of the expression based risk score combined with CAPRA and BMI are used to prognosticate prostate cancer patients. Based on the multivariate Cox regression data, it was hypothesized that a combination of the CAPRA score together with the expression based risk score and BMI will provide a significant improvement in prognostic power over Risk Score alone. To evaluate this hypothesis, a sub-cohort Radical Prostatectomy (RP) patient cohort with ISUP score 1 or 2 was selected and a logistic regression model to combine the expression based risk score with the CAPRA score and BMI to predict the 5-year risk of biochemical recurrence after surgery was generated. The logit(p) regression function was transformed to p=1/(1+e^(-logit(p)) in order to calculate the probability p for an individual patient to experience a biochemical relapse within 5 years after surgery.

Next, this BMI, CAPRA score and expression based risk score logistic regression model was tested independently on ISUP 1 or ISUP 1 or 2 patients who were eligible for statistical data analysis. All patients had a minimum of 60 months of follow-up after operation. The methods used to investigate were (i) Kaplan-Meier survival analysis, (i) ROC curve analysis, as well as (iii) re-classification and decision curve analysis.

The data are plotted in the appended Figures, where:

Figs. 1 and 2 are reference Figures which show results of Kaplan-Meier survival analysis of the time to PSA relapse based on the Risk score (CAPRA and PDE4D5, PDE4D7 and PDE4D9 expression levels) only. Fig. 1 plots ISUP 1 patients and Fig. 2 plots ISUP 1 and 2 patients.

Figs. 3 and 4 show results of Kaplan-Meier survival analysis of the time to PSA relapse based on the Risk score (CAPRA and PDE4D5, PDE4D7 and PDE4D9 expression levels) combined with BMI. Fig. 3 plots ISUP 1 patients and Fig. 4 plots ISUP 1 and 2 patients.

Figs. 5-14 show results of Kaplan-Meier survival analysis of the time to PSA relapse based on the Risk score (CAPRA and PDE4D5, PDE4D7 and PDE4D9 expression levels) when patients are selected based on increasing BMI, where Fig. 5 selection is based on BMI > 20, Fig. 6 selection is based on BMI > 21, Fig. 7 selection is based on BMI > 22, ... and Fig. 14 selection is based on BMI > 29.

Figs. 15 and 16: plot the performance of BCR progression-free survival of the pCAPRA_PDE4D579 model (p -> percentile ranked scores of the CAPRA PDE4D579 model) in patients with ISUP Gleason score 1 (Fig. 15) or ISUP Gleason score 1 or 2 (Fig. 16) on needle biopsy tissue and pCAPRA_PDE4D579<=0.292 (low) or pCAPRA _PDE4D579>0.292 (high) BMI<=26 (BMI_low) or BMI>26 (BMI_high). HR and p-value are given.

Fig. 17 plots an AUROC analysis of pCAPRA&PDE4D579&BMI model vs the CARPA score alone. The AUC is given in parenthesis. The clinical endpoint is 5-year BCR progression-free survival after surgery.

Fig. 18 plots an AUROC analysis of pCAPRA&PDE4D579&BMI, pCAPRA&PDE4D579, CAPRA Score, and PRIAS models in patients with ISUP Gleason score 1 on needle biopsy tissue. The AUC is given in parenthesis. The clinical endpoint is 5-year BCR progression-free survival after surgery.

Fig. 19 plots an AUROC analysis of pCAPRA&PDE4D579&BMI, pCAPRA&PDE4D579, CAPRA Score, and PRIAS models in patients with ISUP Gleason score 1 and 2 on needle biopsy tissue. The AUC is given in parenthesis. The clinical endpoint is 5-year BCR progression-free survival after surgery.

The above data were obtained from diagnostic needle biopsies obtained pre-surgery. The results were independently confirmed based on samples collected from the surgically removed prostate (data not shown).

From these data it can be concluded that the Risk score can be significantly improved for ISUP 1 or 2 prostate cancer patients by including the BMI as a clinical parameter in prognosis. As ISUP 1 or 2 patients are usually recommended active surveillance instead of active treatment, these result may provide an alternative treatment strategy for those patients that are increased risk, particularly those with a high risk score and a high BMI, despite being classified as ISUP 1 or 2. An alternative treatment strategy may be beneficial for this patient group.

## Claims

1. A method for predicting an outcome for a prostate cancer subject with International Society for Urological Pathology (ISUP) score of 1 or 2, the method comprising:

determining expression levels or receiving determined expression levels of PDE4D isoforms PDE4D5, PDE4D7

and PDE4D9 in a sample obtained from the subject;
receiving the Cancer of the Prostate Risk Assessment (CAPRA) score determined for the subject;
determining a risk score based on the PDE4D5, PDE4D7 and PDE4D9 expression levels and the CAPRA score;
wherein the method further comprises receiving or determining the Body Mass Index (BMI) of the subject; and
determining an outcome for the subject based on the risk score and the BMI; wherein the outcome is a favorable outcome or a non-favorable outcome.

**2.** The method according to claim 1 wherein the favorable outcome is low chance of post-surgical disease progression and wherein the non-favorable outcome is high chance of post-surgical disease progression.

**3.** The method according to claim 1 or 2, wherein the sample is a sample obtained from the subject wherein the sample comprises at least one tumor cell, preferably wherein the sample is a tumor sample, a blood sample, a urine sample, or a biopsy such as a needle biopsy.

**4.** The method according to any one of the preceding claims, wherein the PDE4D5, PDE4D7 and PDE4D9 expression levels and the CAPRA score are combined to obtain a risk score using a regression function derived from a population of prostate cancer subjects.

**5.** The method according to any one of the preceding claims, wherein the risk score is a modified CAPRA score for the subject, wherein the PDE4D5, PDE4D7 and PDE4D9 expression levels used to calculate the modified CAPRA score are combined to a value representing the expression score in a predefined range, wherein depending on the value a number of points in the range from 0 to 3 are added to the CAPRA score to obtain the modified CAPRA score.

**6.** The method according to claim 5, wherein the value representing the expression score is determined with a scoring function, based on the PDE4D5, PDE4D7 and PDE4D9 expression level, the scoring function having been derived from gene expression profiles for biological samples of prostate cancer subjects.

**7.** The method according to any one of the preceding claims, wherein the PDE4D5, PDE4D7 and PDE4D9 expression levels are normalized using one or more of the reference genes hypoxanthine phosphoribosyl transferase 1 (HPRT1), Tubulin- Alpha- lb (TUBA1B) pumilio RNA-Binding Family Member (PUM1), and TATA box binding protein (TBP).

**8.** The method according to any one of the preceding claims, wherein the method further comprises proposing a primary treatment for the subject based on the determined outcome.

**9.** The method according to claim 8, wherein the recommended treatment is

active surveillance with de-escalation of monitoring when the risk score is low and the BMI is low or high;
treatment with one or more therapies selected from a GLP-1 receptor agonist, a weight lowering medication, surgery, radiation therapy, focal ablation, androgen hormone reducing therapy using either androgen deprivation therapy or androgen receptor signaling inhibition therapy as a monotherapy when the risk score is high and the BMI is high; or
the treatment comprises treatment with one or more therapies selected from androgen hormone reducing therapy using either androgen deprivation therapy or androgen receptor signaling inhibition therapy or a combination thereof, an PDE4D7 expression increasing therapy, surgery, radiation therapy, chemotherapy, targeted radionuclide therapy, and immunotherapy, when the risk score is high and BMI is low.

**10.** A computer program product comprising instructions which, when the program is executed by a computer, cause the computer to carry out a method comprising:

determining a risk score based on received PDE4D5, PDE4D7 and PDE4D9 expression levels and a received CAPRA score, the expression levels being determined in a sample of a prostate cancer subject with ISUP score of 1 or 2, and the CAPRA score obtained from the subject;
determining an outcome for the subject based on the risk score and a received BMI from the subject;
wherein the outcome is a favorable outcome or a non-favorable outcome.

**11.** Use of a kit to determine an outcome for a prostate cancer subject with ISUP score of 1 or 2, the use comprising

using the kit to determine the expression levels of PDE4D isoforms PDE4D5, PDE4D7 and PDE4D9 in sample

obtained from the subject;
determining a risk score based on the PDE4D5, PDE4D7 and PDE4D9 expression levels and a CAPRA score obtained from the subject;
determining an outcome for the subject based on the risk score and a BMI obtained form the subject;
wherein the kit comprises at least one primer and/or probe for determining the gene expression profile for each of PDE4D5, PDE4D7 and PDE4D9, and
optionally, at least one primer and/or probe for determining the gene expression profile for one or more reference genes selected from the group consisting of: hypoxanthine phosphoribosyl transferase 1 (HPRT1), Tubulin-Alpha- lb (TUBA1B) pumilio RNA-Binding Family Member (PUM1), and TATA box binding protein (TBP); and
optionally, at least one agent for determining a prostate-specific antigen (PSA) level in a biological sample obtained from the subject.

**12.** GLP-1 receptor agonist for use in the treatment of prostate cancer in a subject with ISUP score 1 or 2.

**13.** GLP-1 receptor agonist for use according to claim 12, wherein the use comprises:

determining or receiving the determined expression levels of PDE4D isoforms PDE4D5, PDE4D7 and PDE4D9 in a sample obtained from the subject;
receiving the CAPRA score determined for the subject;
determining a risk score based on the PDE4D5, PDE4D7 and PDE4D9 expression levels and the Cancer of the Prostate Risk Assessment (CAPRA) score;

receiving or determining the Body Mass Index (BMI) of the subject;
wherein the GLP-1 receptor agonist is administered to the subject when the risk score is high and the BMI is high.

**14.** GLP-1 receptor agonist for use according to claim 12 or 13, wherein the GLP-1 receptor agonist is selected from dulaglutide, exenatide, exenatide extended-release, liraglutide, lixisenatide, semagluride injection, emaglutide tablets, albiglutide, and tirzepatide.

## Fig. 1

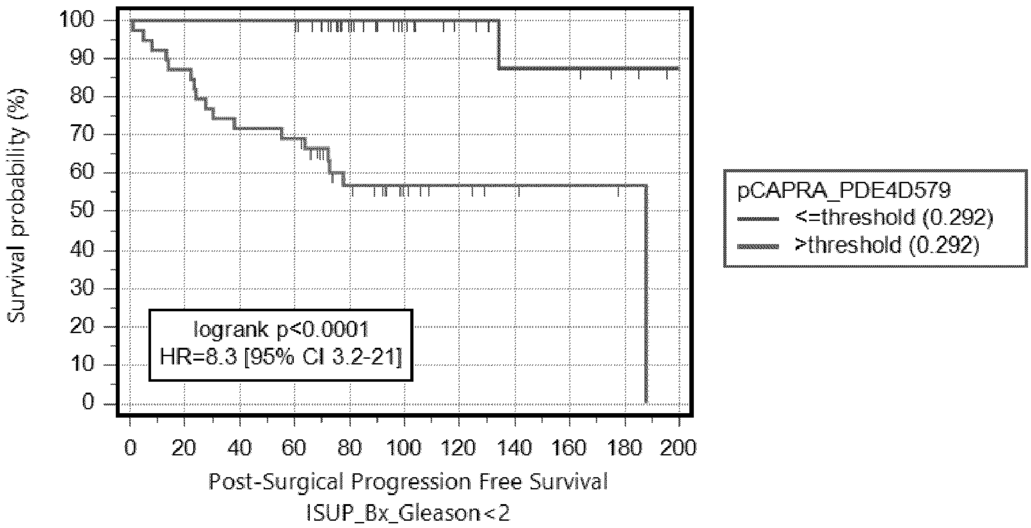


Number at risk

Group: <=threshold (0.292)

36 36 36 36 25 15 10 7 7 5 3

Group: >threshold (0.292)

39 34 28 27 17 10 7 3 2 1 0

## Fig. 2

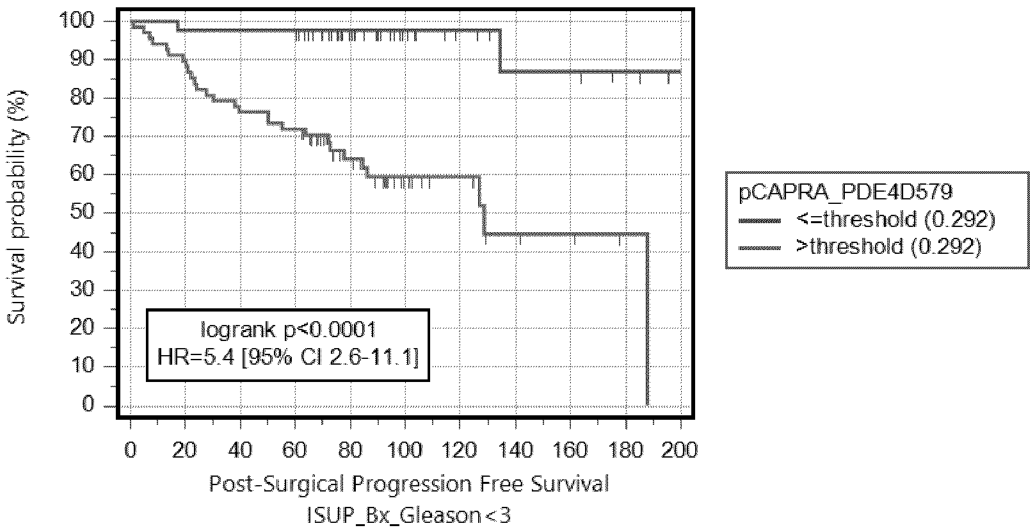


Number at risk

Group: <=threshold (0.292)

44 43 43 43 29 16 11 8 8 6 4

Group: >threshold (0.292)

68 61 52 49 29 14 10 4 3 1 0

**Fig. 3**

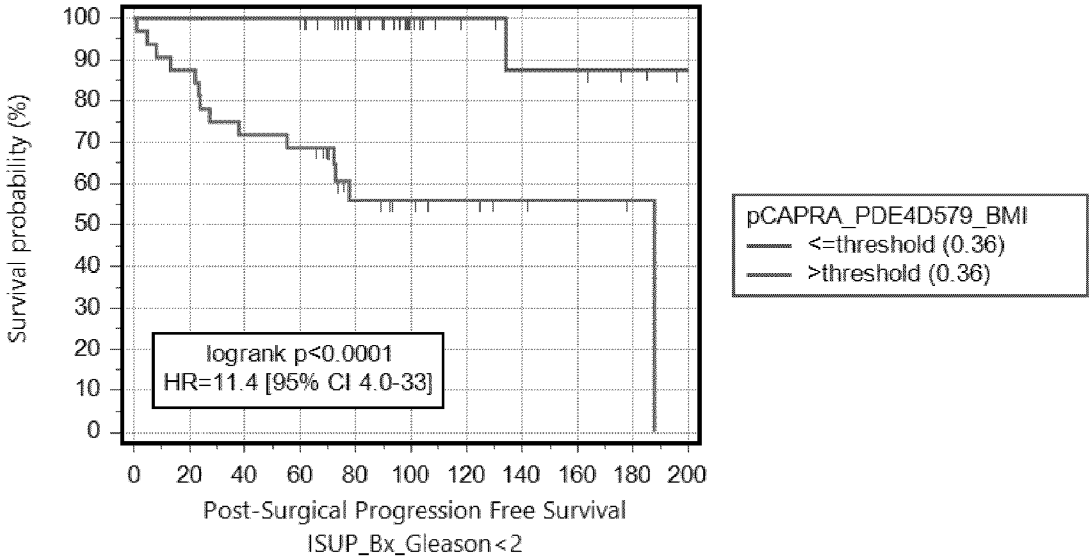


Number at risk

Group: <=threshold (0.36)

38 38 38 38 28 14 9 7 7 5 3

Group: >threshold (0.36)

32 28 23 22 12 9 7 3 2 1 0

**Fig. 4**

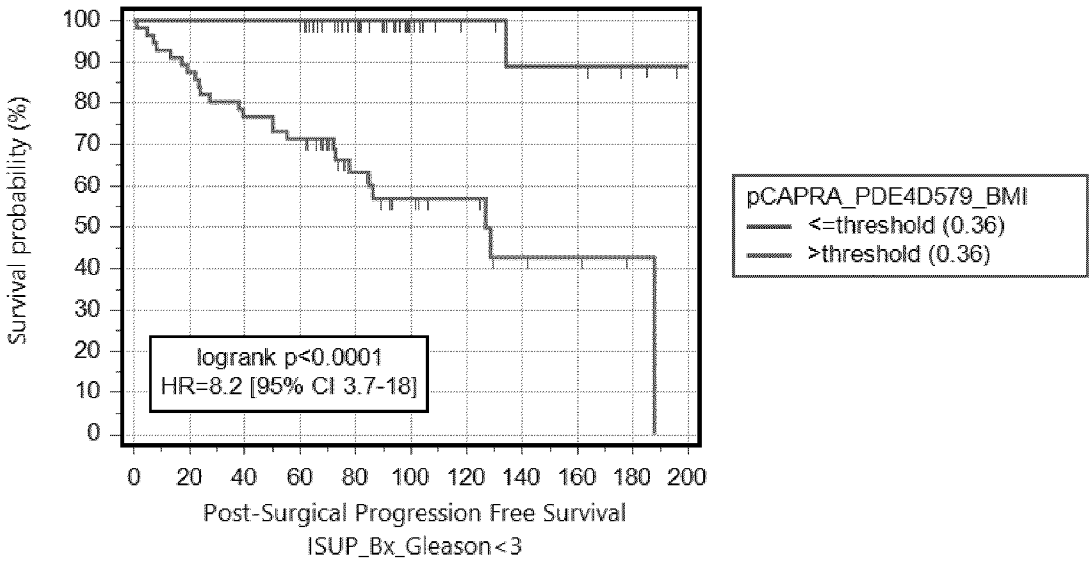


Number at risk

Group: <=threshold (0.36)

48 48 48 48 34 15 10 8 8 6 4

Group: >threshold (0.36)

56 49 43 40 21 13 10 4 3 1 0

**Fig. 5**

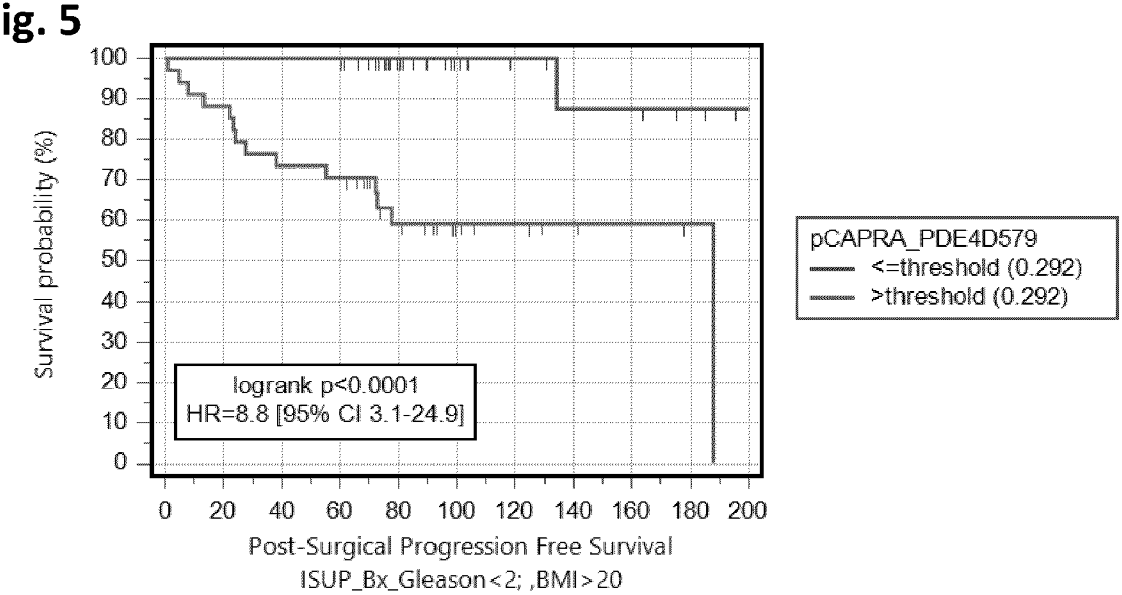


Number at risk

Group: <=threshold (0.292)

34 34 34 34 23 13 9 7 7 5 3

Group: >threshold (0.292)

34 30 25 24 15 9 7 3 2 1 0

**Fig. 6**

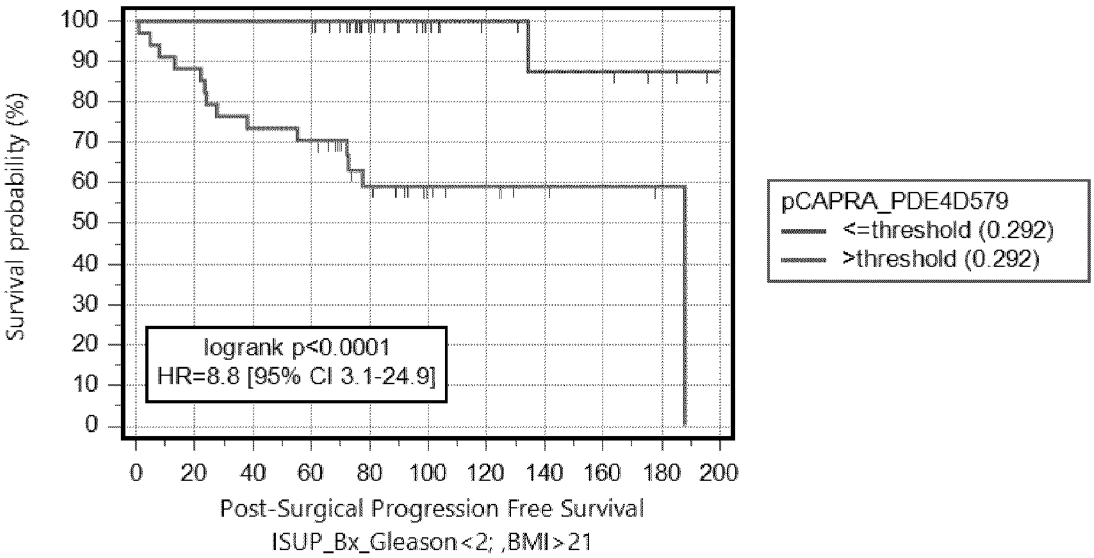


Number at risk

Group: <=threshold (0.292)

34 34 34 34 23 13 9 7 7 5 3

Group: >threshold (0.292)

34 30 25 24 15 9 7 3 2 1 0

**Fig. 7**

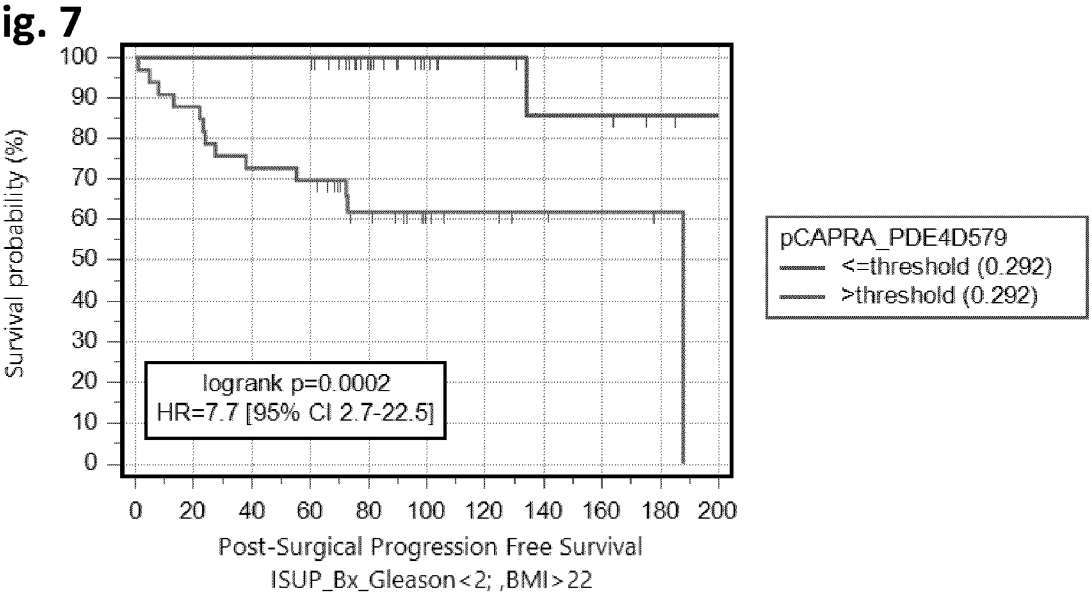


Number at risk

Group: <=threshold (0.292)

31 31 31 31 21 11 8 6 6 4 3

Group: >threshold (0.292)

33 29 24 23 15 9 7 3 2 1 0

**Fig. 8**

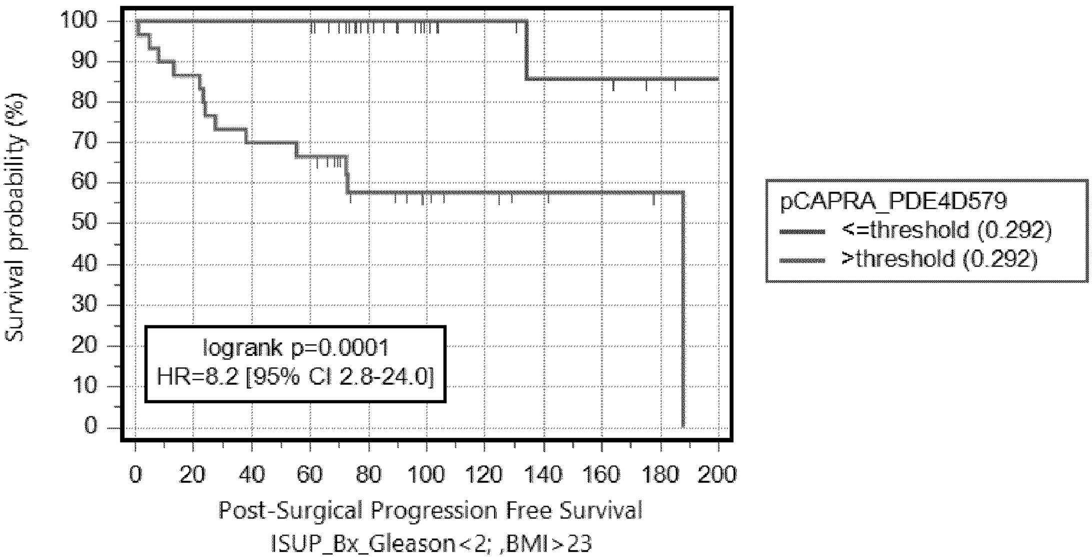


Number at risk

Group: <=threshold (0.292)

29 29 29 29 19 11 8 6 6 4 3

Group: >threshold (0.292)

30 26 21 20 12 9 7 3 2 1 0

## Fig. 9

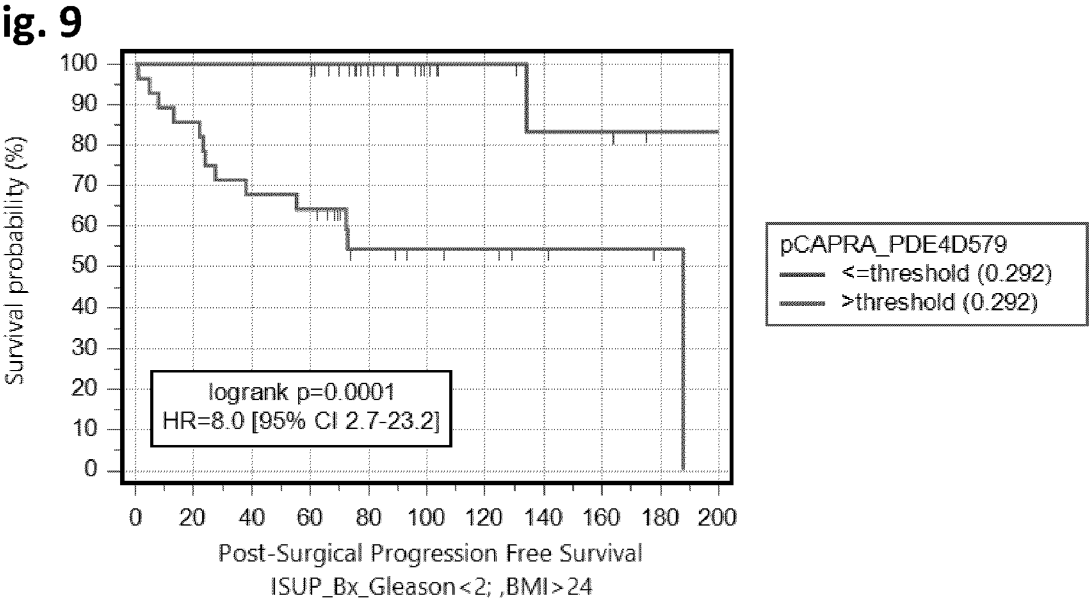


Number at risk
Group: <=threshold (0.292)
26 26 26 26 17 10 7 5 5 3 3
Group: >threshold (0.292)
28 24 19 18 10 8 7 3 2 1 0

## Fig. 10

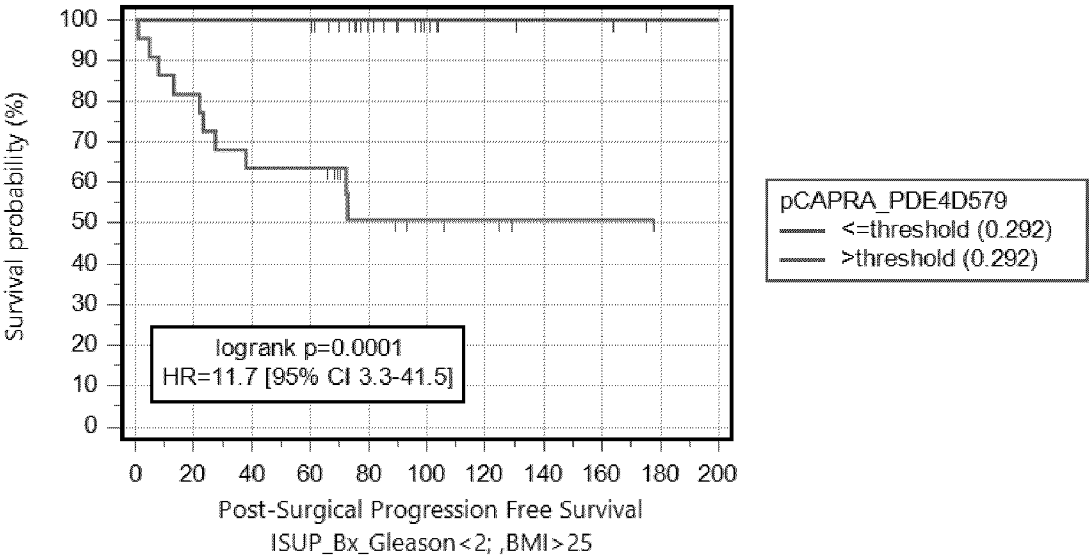


Number at risk
Group: <=threshold (0.292)
24 24 24 24 15 8 5 4 4 2 2
Group: >threshold (0.292)
22 18 14 14 8 6 5 1 1 0 0

Fig. 11

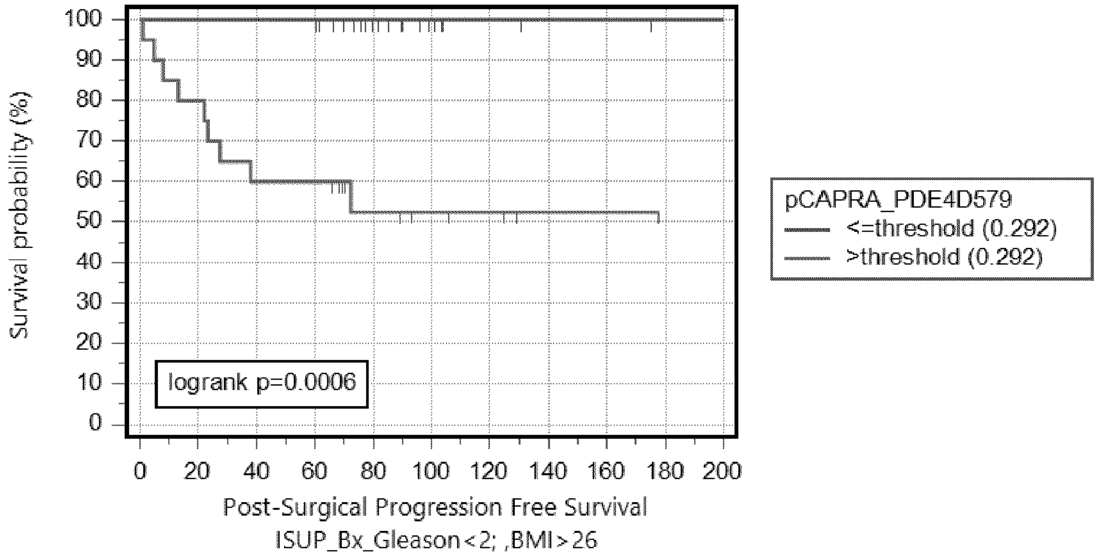


Number at risk

Group: <=threshold (0.292)

20 20 20 20 12 6 3 2 2 1 1

Group: >threshold (0.292)

20 16 12 12 7 5 4 1 1 0 0

Fig. 12

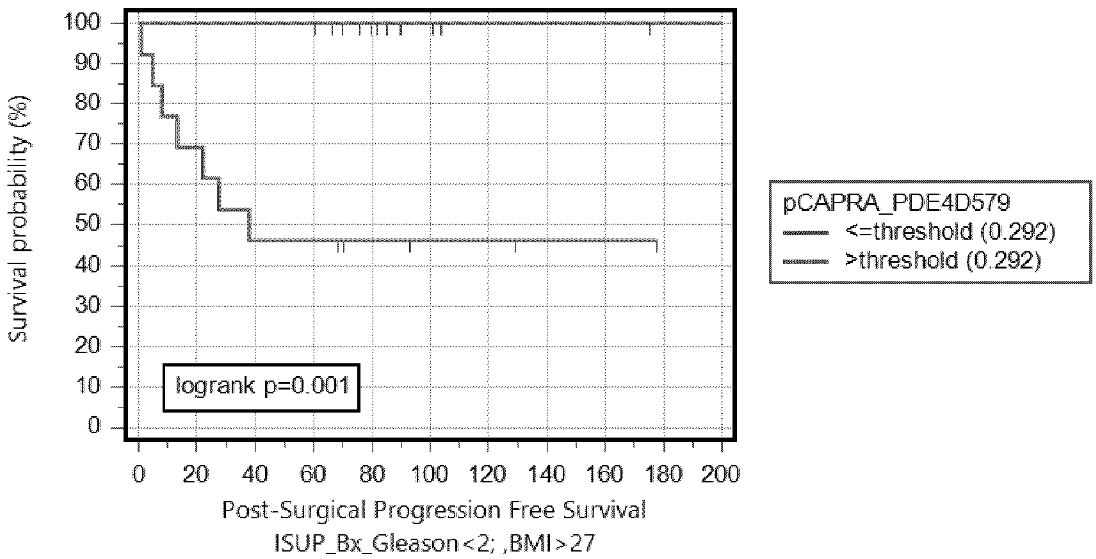


Number at risk

Group: <=threshold (0.292)

14 14 14 14 9 5 2 2 2 1 1

Group: >threshold (0.292)

13 9 6 6 4 3 3 1 1 0 0

**Fig. 13**

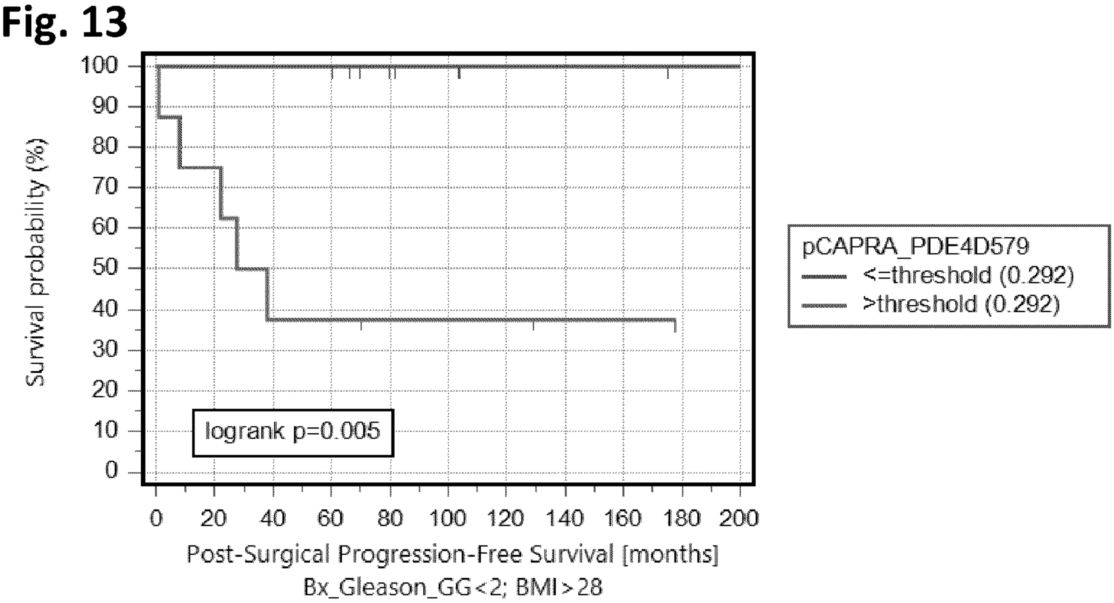


Number at risk

Group: <=threshold (0.292)

9 9 9 9 5 4 2 2 2 1 1

Group: >threshold (0.292)

8 6 3 3 2 2 2 1 1 0 0

**Fig. 14**

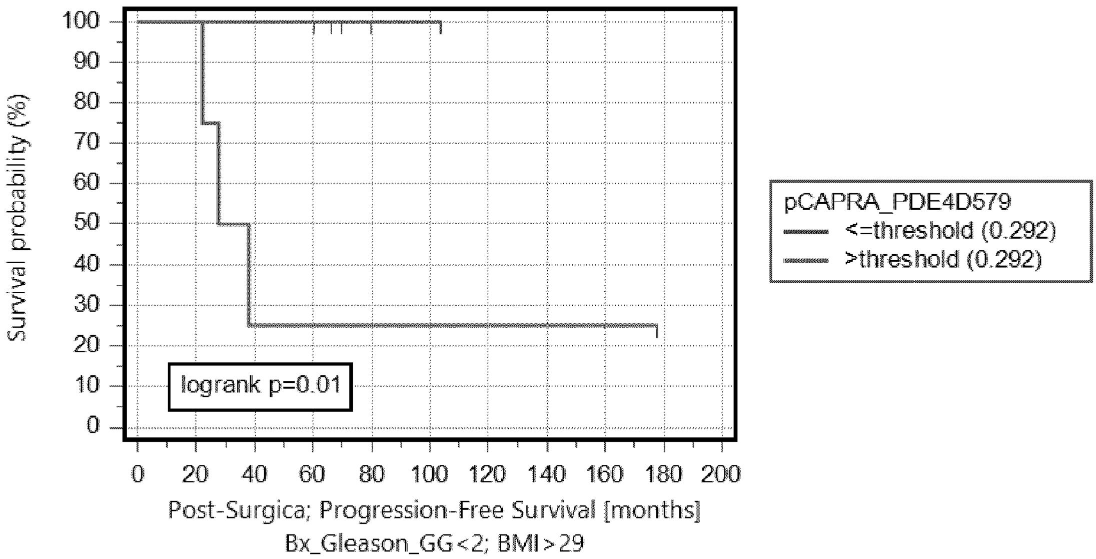


Number at risk

Group: <=threshold (0.292)

6 6 6 6 2 2 0 0 0 0

Group: >threshold (0.292)

4 4 1 1 1 1 1 1 1 0

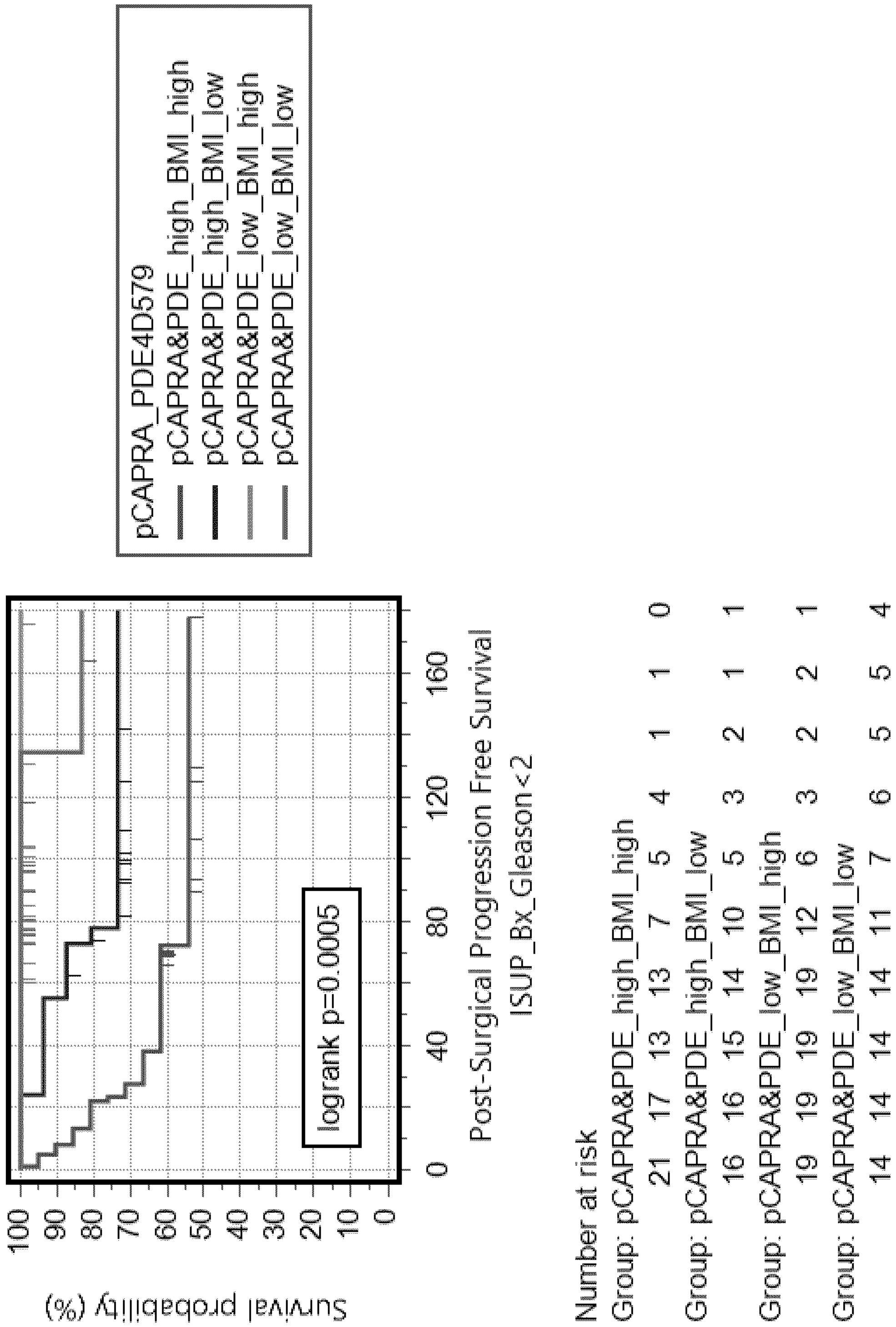
pCAPRA_PDE4D579
pCAPRA&PDE_high_BMI_high
pCAPRA&PDE_high_BMI_low
pCAPRA&PDE_low_BMI_high
pCAPRA&PDE_low_BMI_low
logrank p=0.0005
Survival probability (%)
100 90 80 70 60 50 40 30 20 10 0
0 40 80 120 160
Post-Surgical Progression Free Survival
ISUP_Bx_Gleason<2
Number at risk
Group: pCAPRA&PDE_high_BMI_high
21 17 13 13 7 5 4 1 1 0
Group: pCAPRA&PDE_high_BMI_low
16 16 15 14 10 5 3 2 1 1
Group: pCAPRA&PDE_low_BMI_high
19 19 19 19 12 6 3 2 2 1
Group: pCAPRA&PDE_low_BMI_low
14 14 14 14 11 7 6 5 5 4

**Fig. 15**

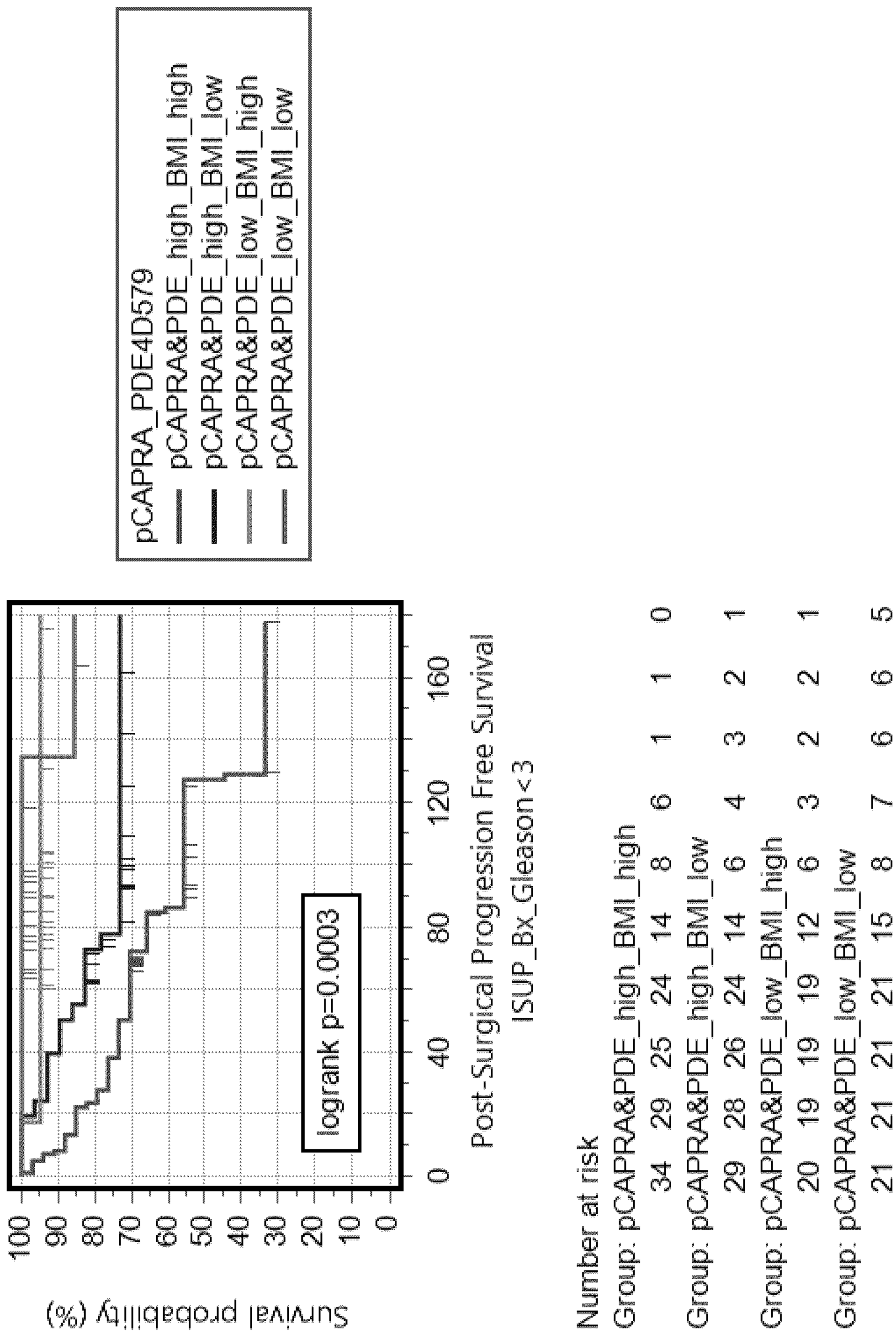
pCAPRA_PDE4D579
pCAPRA&PDE_high_BMI_high
pCAPRA&PDE_high_BMI_low
pCAPRA&PDE_low_BMI_high
pCAPRA&PDE_low_BMI_low
logrank p=0.0003
Survival probability (%)
100 90 80 70 60 50 40 30 20 10 0
0 40 80 120 160
Post-Surgical Progression Free Survival
ISUP_Bx_Gleason<3
Number at risk
Group: pCAPRA&PDE_high_BMI_high
34 29 25 24 14 8 6 1 1 0
Group: pCAPRA&PDE_high_BMI_low
29 28 26 24 14 6 4 3 2 1
Group: pCAPRA&PDE_low_BMI_high
20 19 19 19 12 6 3 2 2 1
Group: pCAPRA&PDE_low_BMI_low
21 21 21 21 15 8 7 6 6 5

Fig. 16

**Fig. 17**

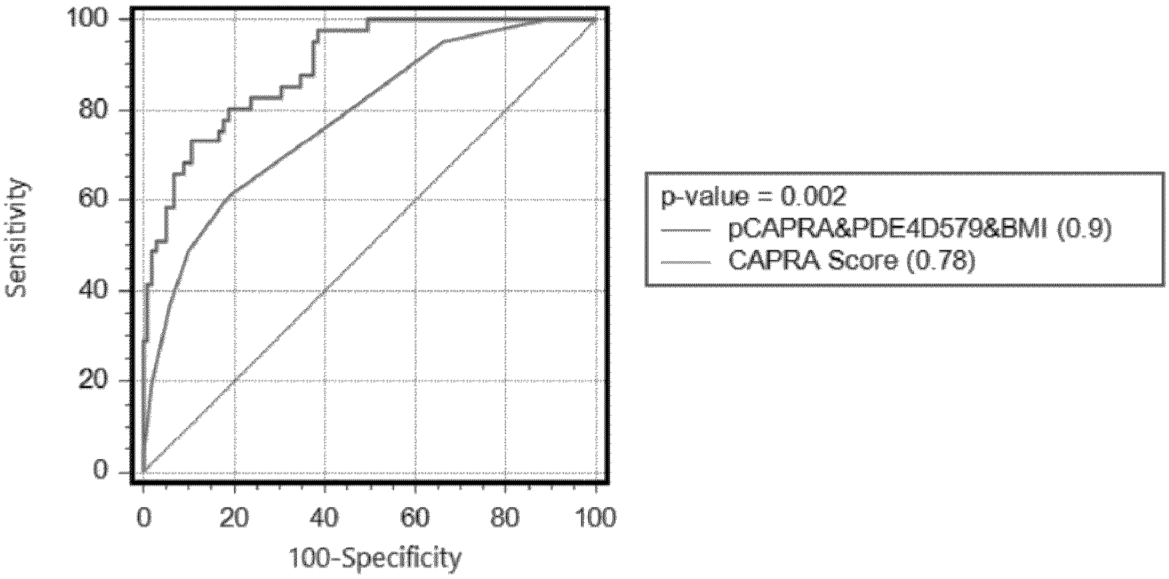
100
80
60
40
20
0
Sensitivity
0
20
40
60
80
100
100-Specificity
p-value = 0.002
pCAPRA&PDE4D579&BMI (0.9)
CAPRA Score (0.78)

**Fig. 18**

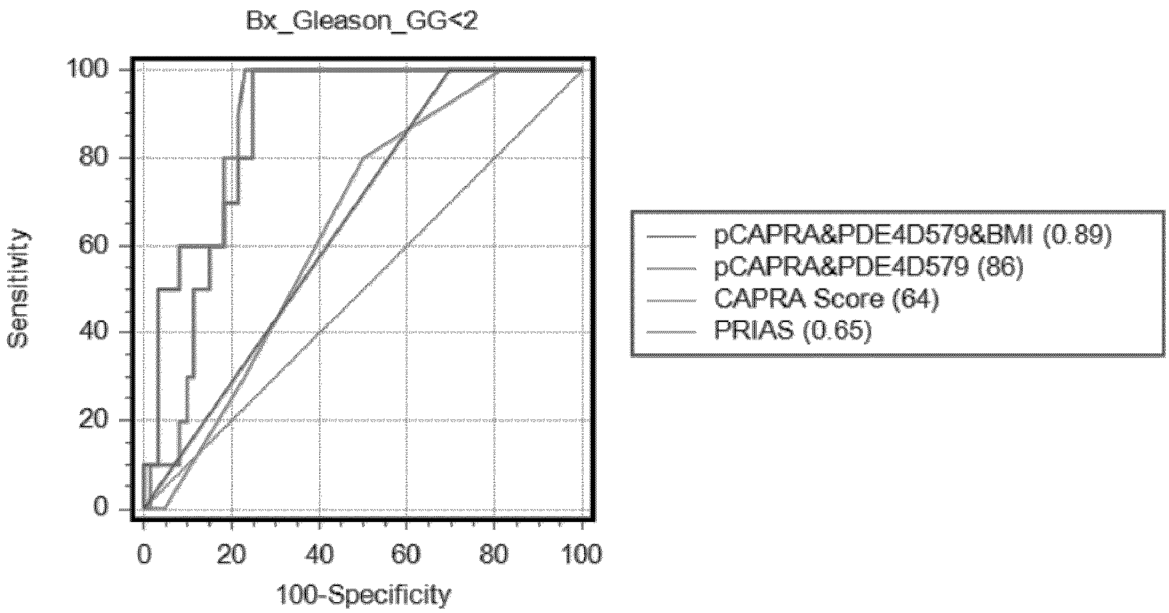
Bx_Gleason_GG<2
100
80
60
40
20
0
Sensitivity
0
20
40
60
80
100
100-Specificity
pCAPRA&PDE4D579&BMI (0.89)
pCAPRA&PDE4D579 (86)
CAPRA Score (64)
PRIAS (0.65)

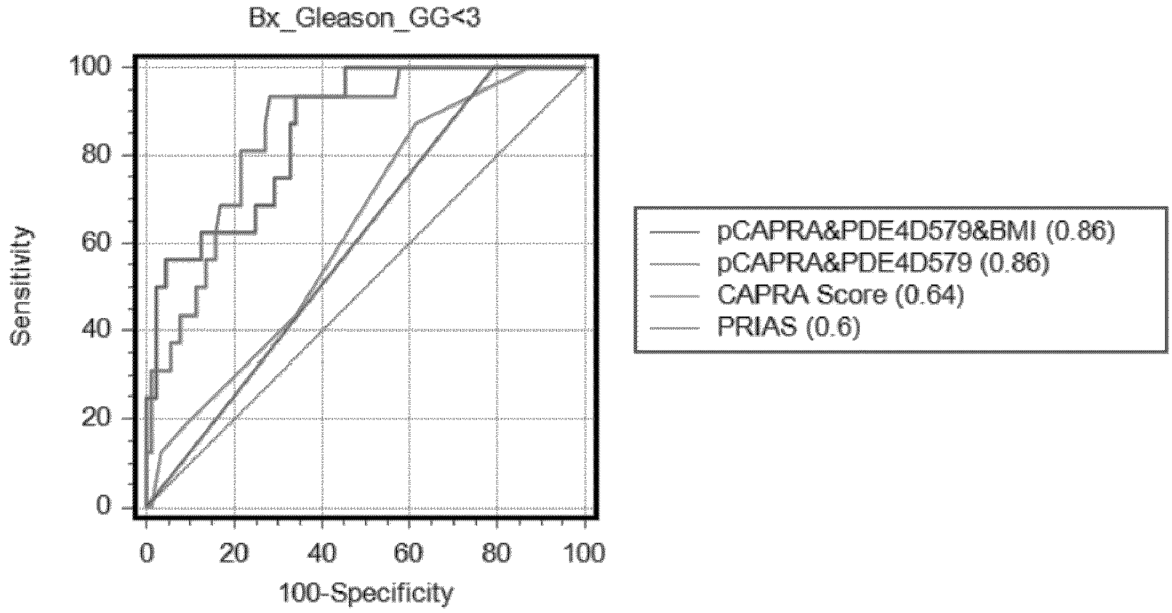
Bx_Gleason_GG<3
Sensitivity
100-Specificity
0
20
40
60
80
100
pCAPRA&PDE4D579&BMI (0.86)
pCAPRA&PDE4D579 (0.86)
CAPRA Score (0.64)
PRIAS (0.6)

Fig. 19

Europäisches Patentamt
European Patent Office
Office européen des brevets

# EUROPEAN SEARCH REPORT

Application Number
EP 24 20 9215

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GULLIVER CHLOE ET AL: "The CAPRA&PDE4D5/7/9 Prognostic Model Is Significantly Associated with Adverse Post-Surgical Pathology Outcomes", CANCERS, vol. 15, no. 1, 30 December 2022 (2022-12-30), page 262, XP093269943, CH ISSN: 2072-6694, DOI: 10.3390/cancers15010262 * the whole document * | 1-14 | INV. C12Q1/6886 |
| A | US 2020/308654 A1 (HOFFMANN RALF DIETER [DE]) 1 October 2020 (2020-10-01) * paragraphs [0162] - [0190] * * claims 1-16 * | 1-14 | |
| A | DIANNE VAN STRIJP ET AL: "The Prognostic PDE4D7 Score in a Diagnostic Biopsy Prostate Cancer Patient Cohort with Longitudinal Biological Outcomes", PROSTATE CANCER, vol. 2018, 26 July 2018 (2018-07-26), pages 1-11, XP055565132, ISSN: 2090-3111, DOI: 10.1155/2018/5821616 * the whole document * | 1-14 | TECHNICAL FIELDS SEARCHED (IPC): C12Q |
| A | GULLIVER CHLOE ET AL: "Reduced PDE4D7 in prostate cancer correlates with genomic downregulation within the upstream PDE4D coding region", FUTURE SCIENCE OA JUN 2017, vol. 9, no. 9, 1 October 2023 (2023-10-01), XP093113261, ISSN: 2056-5623, DOI: 10.2144/fsoa-2023-0064 * the whole document * | 1-14 | |

-/--

The present search report has been drawn up for all claims

3

| Place of search | Date of completion of the search | Examiner |
|---|---|---|
| The Hague | 15 April 2025 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 20 9215

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARCIA ALVES DE INDA ET AL: "Validation of Cyclic Adenosine Monophosphate Phosphodiesterase-4D7 for its Independent Contribution to Risk Stratification in a Prostate Cancer Patient Cohort with Longitudinal Biological Outcomes", EUROPEAN UROLOGY FOCUS, 1 June 2017 (2017-06-01), XP055481303, NL ISSN: 2405-4569, DOI: 10.1016/j.euf.2017.05.010 * the whole document * ----- | 1-14 | |
| A | WO 2024/083612 A1 (KONINKLIJKE PHILIPS NV [NL]) 25 April 2024 (2024-04-25) * the whole document * ----- | 1-14 | |
| X | YU XU ET AL: "Effect of glucagon-like peptide-1 receptor agonists on prostate cancer: A review", MEDICINE, vol. 103, no. 41, 11 October 2024 (2024-10-11), page e39956, XP093270180, ISSN: 1536-5964, DOI: 10.1097/MD.0000000000039956 * the whole document * ----- | 12,14 | TECHNICAL FIELDS SEARCHED (IPC) |

-/--

The present search report has been drawn up for all claims

| Place of search | Date of completion of the search | Examiner |
|---|---|---|
| The Hague | 15 April 2025 | Bruma, Anja |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 20 9215

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LAVALETTE CÉLINE ET AL: "Body mass index trajectories and prostate cancer risk: Results from the EPICAP study", CANCER MEDICINE, vol. 9, no. 17, 8 July 2020 (2020-07-08), pages 6421-6429, XP093269942, GB ISSN: 2045-7634, DOI: 10.1002/cam4.3241 Retrieved from the Internet: URL:https://onlinelibrary.wiley.com/doi/full-xml/10.1002/cam4.3241> * the whole document * ----- | 1-14 | |
| A | DE COBELLI OTTAVIO ET AL: "Body mass index was associated with upstaging and upgrading in patients with low-risk prostate cancer who met the inclusion criteria for active surveillance", UROLOGIC ONCOLOGY: SEMINARS AND ORIGINAL INVESTIGATIONS, vol. 33, no. 5, 1 May 2015 (2015-05-01), pages 201.e1-201.e8, XP093269963, AMSTERDAM, NL ISSN: 1078-1439, DOI: 10.1016/j.urolonc.2015.02.004 * the whole document * ----- | 1-14 | TECHNICAL FIELDS SEARCHED (IPC) |

The present search report has been drawn up for all claims

| Place of search | Date of completion of the search | Examiner |
|---|---|---|
| The Hague | 15 April 2025 | Bruma, Anja |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 24 20 9215

This annex lists the patent family members relating to the patent documents cited in the above-mentioned European search report. The members are as contained in the European Patent Office EDP file on
The European Patent Office is in no way liable for these particulars which are merely given for the purpose of information.

15-04-2025

| Patent document cited in search report | Publication date | Patent family member(s) | Publication date |
|---|---|---|---|
| US 2020308654 A1 | 01-10-2020 | CN 111742061 A | 02-10-2020 |
| | | DK 3728640 T3 | 09-05-2022 |
| | | EP 3502280 A1 | 26-06-2019 |
| | | EP 3728640 A1 | 28-10-2020 |
| | | ES 2911245 T3 | 18-05-2022 |
| | | JP 7336442 B2 | 31-08-2023 |
| | | JP 2021506326 A | 22-02-2021 |
| | | US 2020308654 A1 | 01-10-2020 |
| | | WO 2019122037 A1 | 27-06-2019 |
| WO 2024083612 A1 | 25-04-2024 | EP 4357462 A1 | 24-04-2024 |
| | | WO 2024083612 A1 | 25-04-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019122037 A1
- WO 2019122037 A

### Non-patent literature cited in the description


- GLOBOCAN. **FERLAY J et al.** Cancer Incidence and Mortality Worldwide: IARC CancerBase No. 11 [Internet. International Agency for Research on Cancer, 2012, vol. v1.0
- **RODRIGUES G. et al.** Pre-treatment risk stratification of prostate cancer patients: A critical review. *Canadian Urological Association Journal*, 2012, vol. 6 (2), 121-127
- **MOHLER J et al.** NCCN clinical practice guidelines in oncology: Prostate cancer, Version 1.2016. *Journal of the National Comprehensive Cancer Network*, 2016, vol. 14 (1), 19-30
- **HERNANDEZ D.J. et al.** Contemporary evaluation of the D'Amico risk classification of prostate cancer. *Journal of Urology*, 2007, vol. 70 (5), 931-935
- **JUNG J.W. et al.** Stratification of patients with intermediate-risk prostate cancer. *BJU International*, 2015, vol. 115 (6), 907-912
- **ABERN M.R. et al.** Delayed radical prostatectomy for intermediate-risk prostate cancer is associated with biochemical recurrence: Possible implications for active surveillance from the SEARCH database. *The Prostate*, 2013, vol. 73 (4), 409-417
- **SAMBROOK et al.** Molecular Cloning. A Laboratory Manual. Cold Spring Harbor Laboratory Press, 1989
- **AUSUBEL et al.** Current Protocols in Molecular Biology. John Wiley & Sons, 1987
- Methods in Enzymology. Academic Press
- Computational Molecular Biology. Oxford University Press, 1988
- Biocomputing: Informatics And Genome Projects. Academic Press, 1993
- Computer Analysis Of Sequence Data. Humana Press, 1994
- **VON HEINJE, G**. Sequence Analysis In Molecular Biology. Academic Press, 1987
- Sequence Analysis Primer;. M Stockton Press, 1991
- **CARILLO, H** ; **LIPTON, D**. *SIAM J. Applied Math*, 1988, vol. 48, 1073
- Guide To Huge Computers. Academic Press, 1994
- **CARILLO, H** ; **LIPTON, D**. *Siam J. Applied Math*, 1988, vol. 48, 1073
- **DEVEREUX, J et al.** *Nucleic Acids Research*, 1984, vol. 12 (1), 387
- **ATSCHUL, S. F et al.** *J. Molec. Biol*, 1990, vol. 215, 403
- **LUGHEZANI G. et al.** Predictive and prognostic models in radical prostatectomy candidates: A critical analysis of the literature. *European Urology*, 2010, vol. 58 (5), 687-700
- **COOPERBERG M.R**. The UCSF Cancer of the Prostate Risk Assessment (CAPRA) Score: A straightforward and reliable pre-operative predictor of disease recurrence after radical prostatectomy. *Journal of Urology*, 2005, vol. 173 (6), 1938-1942
- **BRAJTBORD J.S. et al.** The CAPRA score at 10 years: Contemporary perspectives and analysis of supporting studies. *European Urology*, 2017, vol. 71 (5), 705-709
- **EPSTEIN et al.** Contemporary Prostate Cancer Grading System: A Validated Alternative to the Gleason Score. *Eur Urol.*, March 2016, vol. 69 (3), 428-35
- **RAMAMOORTH M** ; **NARVEKAR A**. Non viral vectors in gene therapy- an overview.. *J Clin Diagn Res.*, January 2015, vol. 9 (1), GE01-6
- **BLAIR et al.** Measuring cAMP Specific Phosphodiesterase Activity: A Two-step Radioassay. *Bio Protoc*, 05 April 2020, vol. 10 (7), e3581
- **CHEN et al.** *Acta Pharmacol Sin*, 2017, vol. 38, 1205-1235
- **VAN STRIJP et al.** *Prostate Cancer*, vol. 2018 (1)
- **TAYLOR et al.** *Cancer Cell*, 2010, vol. 18 (1), 11-22